# EUROPEAN PATENT APPLICATION

(11) **EP 4 233 837 A1**
(43) Date of publication of application: **30.08.2023**
(21) Application number: 22158573.0
(22) Date of filing: 24.02.2022
(51) Int. Cl.: A61K 9/00, A61K 9/08, A61K 31/7048, A61K 47/02, A61K 47/10, A61K 47/12, A61K 47/26, A61P 1/16, A61P 31/00

(54) **SOLID AND ORAL ETOPOSIDE TONIRIBATE COMPOSITIONS**

(71) Applicant: CellAct Pharma GmbH, 44227 Dortmund (DE)
(72) Inventor: The designation of the inventor has not yet been filed
(74) Representative: Kuttenkeuler, David

(57) **Abstract**

The present invention relates to new stable oral pharmaceutical formulations of etoposide toniribate, lyophilized composition of etoposide toniribate solutions and methods of treating cancer using said formulations and compositions.

## Description

### FIELD

The present invention relates to new stable oral pharmaceutical formulations of etoposide toniribate, lyophilized composition of etoposide toniribate solutions and methods of treating cancer using said formulations and compositions.

### BACKGROUND

Etoposide is a chemotherapeutic drug derived from podophyllotoxin and acts as an inhibitor of topoisomerase II. The enzyme topoisomerase II induces transient DNA double strand breaks to enable modifications of DNA tertiary structure. Etoposide acts as a topoisomerase II poison leading to a stabilization of the cleavable complex, resulting in multiple non- repairable double strand breaks.

Etoposide toniribate is an analogue and/or pro-drug of etoposide, differing from etoposide in that a water-soluble ester group is attached to etoposide. Etoposide toniribate corresponds to a compound according to formula I:

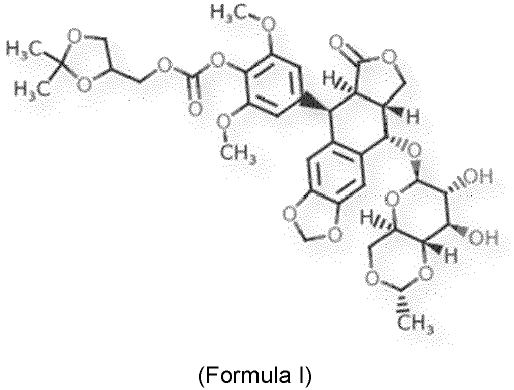

Etoposide toniribate (also known as Cap7.1 or EDO-S7.1) is effective to treat cancer, including metastatic tumours, tumours reducing an organ function, and/or in patients having advanced, progressive cancer or an end-stage cancer. The efficacy of etoposide toniribate in the treatment of cancer is reported in, for example: Keilholz U et al. First-in-man dose escalation and pharmacokinetic study of CAP7.1, a novel prodrug of etoposide, in adults with refractory solid tumours. Eur J Cancer. 2017 Jul;80:14-25; and Pape UF et al. Randomized, multicenter phase II trial of CAP7.1 in patients with advanced biliary tract cancers. J. Clin. Oncol; 2016, 34:4_suppl, 441-441, each of which is incorporated herein by reference.

While etoposide toniribate is an effective anti-cancer therapy, to date it has proven to be difficult to formulate. Currently, etoposide toniribate is formulated as a powder that must be stored at -70 Celsius. The powder must be solubilised in polyethoxylated castor oil (e.g. Cremophor EL^{®}) and ethanol (50:50 oil to ethanol) in glass vials, and then subsequently diluted in sodium chloride (0.9%) for infusion. This is inappropriate for large scale production of an etoposide toniribate formulation, and the reconstitution process is cumbersome and time-consuming. There is therefore a need for new stable formulations of etoposide toniribate.

Amongst the advantages of the present invention is that it provides a solution to this problem in the art.

### SUMMARY OF INVENTION

The present invention provides a new liquid formulation of etoposide toniribate that is stable at refrigerator temperature (2-8°C), and room temperature (20-25°C) for at least 3 months, and is still stable even at higher temperatures. The pharmaceutical formulations of the invention can be directly diluted to form an infusion solution suitable for administration to a patient or can be, directly administered as oral dosage form. Further lyophilized composition of etoposide toniribate is provided which can be resuspended as Infusion solutions prepared from pharmaceutical formulations according to the invention which are also stable at room temperature. The invention thus provides a formulation and compositions that is easier to store and handle in comparison to known formulations of etoposide toniribate.

The invention further provides a liquid formulation of etoposide toniribate in an oral dosage form. In an alternative aspect, the invention further provides solid etoposide toniribate formulations, such as a lyophilized composition of etoposide toniribate. The aspects of the invention in addition provide their use in methods of treating a patient by administering such compositions in a therapeutically effective amount to a subject in need of the treatment, in particular wherein the treatment comprises an oral administration of the compositions and formulations of the invention. Furthermore, the invention herein provides a solid etoposide toniribate composition which is used for packaging and transport that can be resuspended immediately before intravenous administration to a subject.

Accordingly, in a first aspect the invention provides a liquid or solid pharmaceutical formulation, in some embodiments preferably formulated for oral administration, comprising:
etoposide toniribate;
a polysorbate; and
optionally, physiological acceptable organic solvent, such as Ethanol.

In another aspect the invention provides a liquid or solid pharmaceutical formulation, in some embodiments preferably formulated for oral administration, comprising:
Etoposide toniribate;
PEG;
a polysorbate;
optionally, physiological acceptable organic solvent, such as Ethanol;
Benzyl alcohol.

In certain preferred embodiments, the formulation comprises etoposide toniribate at a concentration in the range of from 10 mg/ml to 20 mg/ml.

A physiological acceptable organic solvent is preferably an alcohol or aliphatic alcohol, and in certain embodiments is preferably ethanol.

In certain preferred embodiments, the formulation comprises etoposide toniribate at a concentration in the range of from 40 mg/ml to 100 mg/ml, preferably 50 mg/ml to 100 mg/ml. In certain preferred embodiments, the formulation comprises etoposide toniribate at a concentration of 50 mg/ml or 91 mg/ml.

The term "polysorbate" is employed for its art-recognized meaning, i.e., polyoxyethylene sorbitan fatty acid esters as disclosed and defined in the Handbook Of Pharmaceutical Excipients, edited by Ainley Wade and Paul Weller, The Pharmaceutical Press, London, 1994. Useful polysorbates include polysorbate 20, 21, 40, 60, 61, 65, 80, 81, 85, and 120. Polysorbate 80 is preferred.

In certain preferred embodiments, the PEG is PEG 200-600. In certain preferred embodiments, the PEG is PEG 200-400. In preferred embodiments, the PEG is selected from PEG 200, PEG 300, and PEG 400. In certain preferred embodiments, the PEG in the formulation is PEG 300.

In certain embodiments, the formulation further comprises citric acid.

In certain preferred embodiments, the polysorbate is polysorbate 80.

In a preferred embodiment, the formulation is non-aqueous.

In certain embodiments, the formulation has a pH in the range of from pH 3 to 5, preferably pH 3 to 4. In certain embodiments the formulation has a pH in the range of from pH 3.5 to 4.0. In certain embodiments, the formulation has a pH of 3.7.

In certain embodiments, the formulation has a pH in the range of from pH 5 to 8, preferably from pH 5.0 to 7.8. In certain embodiments, the formulation has a pH in the range of from 5.0 to 7.0, optionally from pH 5.0 to 6.0, or from pH 6.0 to 7.0. In certain alternative preferred embodiments, the formulation has a pH in the range of from 7.3 to 7.8.

In a further aspect is provided a method of preparing an infusion solution comprising diluting a pharmaceutical formulation according to the invention in a diluent. In a further aspect is provided an infusion solution prepared according to such a method.

In preferred embodiments, the diluent is selected from water for injection; 5% glucose solution; 0.45% NaCl saline and 0.9% NaCl saline. In preferred embodiments, the diluent is water for injection.In preferred embodiments, the infusion solution has a concentration of etoposide toniribate in the range of from of from 1 mg/ml to 10 mg/ml, preferably in the range of from 1 mg/ml to 5 mg/ml. Preferably the concentration of etoposide toniribate is 3.1 mg/ml.

In another preferred embodiment, the infusion solution has a concentration of etoposide toniribate in the range of from of from 0.5 mg/ml to 0.8 mg/ml. Preferably the concentration of etoposide toniribate is 0.5 mg/ml or 0.8 mg/ml.

In a further aspect is provided a kit comprising: a pharmaceutical formulation according to the invention; and a diluent. In preferred embodiments, the diluent is selected from water for injection; 5% glucose solution; 0.45% NaCl saline and 0.9% NaCl saline. In preferred embodiments, the diluent is water for injection.

In another aspect is provided a kit comprising: a pharmaceutical formulation according to the invention; and a diluent. In preferred embodiments, the diluent is selected from water for injection; 5% glucose solution; and 0.9% NaCl saline. In preferred embodiments, the diluent is water for injection.

In a further aspect is provided a pharmaceutical formulation or an infusion solution according to the invention, for use in therapy.

In a further aspect is provided a pharmaceutical formulation or an infusion solution according to the invention, for use in a method of treating cancer.

In a further aspect is provided a pharmaceutical formulation or an infusion solution according to the invention, for use in a method of treating biliary tract cancer

In a further aspect is provided a method of treating cancer in a patient in need thereof, the method comprising administering to the patient an effective amount of a pharmaceutical formulation or an infusion solution according to the invention.

In preferred embodiments of all aspects of the invention, the pharmaceutical formulation comprises:
50 mg/ml Etoposide toniribate;
750 mg/ml Polysorbate 80; and
250 mg/ml physiological acceptable organic solvent, such as ethanol.

In preferred embodiments of all aspects of the invention, the pharmaceutical formulation comprises:
91 mg/ml Etoposide toniribate;
631 mg/ml Polysorbate 80; and
252 mg/ml physiological acceptable organic solvent, such as ethanol.

In preferred embodiments of all aspects of the invention, the pharmaceutical formulation comprises:
10 mg/ml Etoposide toniribate;
650 mg/ml PEG 300;
80 mg/ml Polysorbate 80;
242 mg/ml physiological acceptable organic solvent, such as ethanol; and
31 mg/ml Benzyl alcohol.

In preferred embodiments of all aspects of the invention, the pharmaceutical formulation comprises:
20 mg/ml Etoposide toniribate;
650 mg/ml PEG 300;
80 mg/ml Polysorbate 80;
242 mg/ml ethanol; and
31 mg/ml Benzyl alcohol.

In preferred embodiments, the invention provides the above pharmaceutical formulation for oral administration, such as oral dosage form selected from a gel, pill, tablet, caplet, hard capsule or soft capsule.

### DETAILED DESCRIPTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

As used herein, "Etoposide toniribate" and "Cap7.1" are used interchangeably to refer to a compound according to formula I:

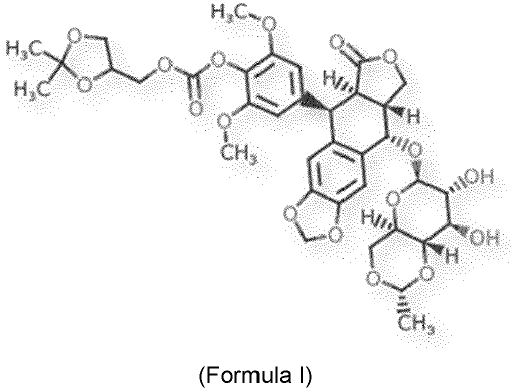

Chemotherapeutic treatment of cancer with etoposide toniribate requires appropriate formulation of the compound. To date, etoposide toniribate has been formulated as a dry powder, which must be stored at -70°C, thereby placing significant demands for maintenance of a reliable storage and distribution cold-chain.

For administration, the powder is dissolved and reconstituted in polyethoxylated castor oil (e.g. Cremophor EL^{®}) and ethanol (at a ratio of 50:50 oil to ethanol), and then subsequently diluted in sodium chloride (0.9%) for infusion.

The present invention provides new, in particular new dry lyophilized, formulations of etoposide toniribate such that the drug can be effectively and stably stored and can be efficiently prepared for administration to patients.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

Accordingly, in a first aspect the invention provides a liquid pharmaceutical formulation, preferably for oral administration, comprising:
etoposide toniribate;
a polysorbate; and
physiological acceptable organic solvent, such as ethanol.

In another aspect the invention provides a liquid pharmaceutical formulation comprising:
Etoposide toniribate;
PEG 300;
a polysorbate;
physiological acceptable organic solvent, such as ethanol; and
Benzyl alcohol.

As demonstrated in the accompanying Examples, a formulation comprising these ingredients shows long-term stability at a range of temperatures, from refrigerator temperature to 40°C. The formulation can also easily be diluted to prepare an infusion solution for administration to a patient. Infusion solutions prepared from the pharmaceutical formulations of the invention also show particularly favourable stability at room temperature, as shown in the Examples. The formulation is in most preferred embodiments provided as oral formulation.

As will be appreciated by the skilled person, "pharmaceutical formulation" in this context refers to a formulation suitable for administration to a patient, and is used interchangeably with "formulation" in this description.

In preferred embodiments the concentration of etoposide toniribate in the formulation is in the range of from 1 mg/ml to 50 mg/ml. Preferably the concentration of etoposide toniribate in the formulation is in the range of from 5 mg/ml to 40 mg/ml, preferably 5 mg/ml to 30 mg/ml. More preferably, the concentration of etoposide toniribate in the formulation is in the range of from 10 mg/ml to 20 mg/ml.

In certain preferred embodiments the concentration of etoposide toniribate in the formulation is 10 mg/ml. In certain preferred embodiments the concentration of etoposide toniribate in the formulation is 20 mg/ml.

In another preferred embodiments the concentration of etoposide toniribate in the formulation is in the range of from 25 mg/ml to 150 mg/ml. Preferably the concentration of etoposide toniribate in the formulation is in the range of from 30 mg/ml to 140 mg/ml, preferably 40 mg/ml to 130 mg/ml, preferably 40 mg/ml to 110 mg/ml. More preferably, the concentration of etoposide toniribate in the formulation is in the range of from 50 mg/ml to 100 mg/ml, preferably 50 mg/ml to 91 mg/ml.

In certain preferred embodiments the concentration of etoposide toniribate in the formulation is 50 mg/ml. In certain preferred embodiments the concentration of etoposide toniribate in the formulation is 91 mg/ml.

The pharmaceutical formulation of the invention comprises a polysorbate. Polysorbates are surfactants derived from PEGylated sorbitan (a derivative of sorbitol) esterified with fatty acids, for example oleic acid. Polysorbates include Polysorbate 80 and Polysorbate 20, also known as Tween 80 and Tween 20, respectively. In certain embodiments the polysorbate in the formulation is selected from polysorbate 80 and polysorbate 20. In preferred embodiments of the pharmaceutical formulation of the present invention the polysorbate is Polysorbate 80 (polyoxyethylene-sorbitan(20)-monooleate).

In certain preferred embodiments, the concentration of the polysorbate (preferably Polysorbate 80) in the formulation is in the range of from 500 mg/ml to 1000 mg/ml. Preferably, the polysorbate is at a concentration in the range of from 600 mg/ml to 800 mg/ml, more preferably 630 mg/ml to 800 mg/ml. In a preferred embodiment, the polysorbate (e.g. polysorbate 80) is present in the formulation at a concentration in the range of from 630 mg/ml to 750 mg/ml. Preferably the polysorbate is present at a concentration of 750 mg/ml. Alternatively preferably, the polysorbate is present at a concentration of 631 mg/ml.

In another preferred embodiments, the concentration of the polysorbate (preferably Polysorbate 80) in the formulation is in the range of from 50 mg/ml to 100 mg/ml. Preferably, the polysorbate is at a concentration in the range of from 60 mg/ml to 100mg/ml, more preferably 60 mg/ml to 90 mg/ml. In a preferred embodiment, the polysorbate is present in the formulation at a concentration in the range of from 70 mg/ml to 90 mg/ml, more preferably in the range of from 75 mg/ml to 85 mg/ml. Preferably the polysorbate is present at a concentration of 80 mg/ml.

Most preferably the polysorbate is polysorbate 80 and is present in the formulation at a concentration of 80 mg/ml.

Most preferably the polysorbate is polysorbate 80 and is present in the formulation at a concentration of 750 mg/ml.

The pharmaceutical formulation of the invention comprises PEG 200-600 - that is, polyethylene glycols having an average molecular weight in the range of from 200 to 600 g/mol. The PEG used in the formulations of the invention have the general formula: 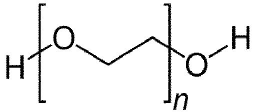 where "n" is in the range of from 4 to 12. For example, "n" may be 4, 5, 6, 7, 8, 9, 10, 11 or 12.

The skilled person will appreciate that for PEG used in the formulations of the invention, the molecular weight or number of repeat units "n" refers to the average for the particular PEG used.

In certain preferred embodiments, the PEG is PEG 200-400. In preferred embodiments, the PEG is selected from PEG 200, PEG 300, and PEG 400.

In certain preferred embodiments, the PEG in the formulation is PEG 300. That is, polyethylene glycols having the general formula: 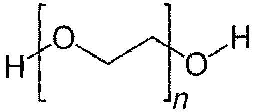 where "n" is 6. As would be appreciated by the skilled person, PEG 300 is also known as PEG 6, macrogol 300 or polyglycol 300.

In preferred embodiments, the concentration of PEG (e.g. PEG 300) in the formulation is in the range of from 100 mg/ml to 1000 mg/ml. Preferably the concentration of PEG in the formulation is in the range of from 500 mg/ml to 1000 mg/ml. More preferably, the concentration of PEG, preferably PEG 300, in the formulation is in the range of from 500 mg/ml to 700 mg/ml, more preferably 600 mg/ml to 700 mg/ml.

In a most preferred embodiment the concentration of PEG in the pharmaceutical formulation is 650 mg/ml. Preferably the PEG is PEG 300.

The pharmaceutical formulation of the invention comprises ethanol - that is, 100% ethanol. 100% ethanol is also known as dehydrated alcohol, anhydrous ethanol, or absolute ethyl alcohol.

In preferred embodiments, the concentration of dehydrated alcohol in the formulation is in the range of from 10 mg/ml to 1000 mg/ml. Preferably the concentration of dehydrated alcohol in the formulation is in the range of from 50 mg/ml to 500 mg/ml. More preferably, the concentration of dehydrated alcohol in the formulation is in the range of from 100 mg/ml to 400 mg/ml, more preferably 200 mg/ml to 300 mg/ml. In a further preferred embodiment, the concentration of dehydrated alcohol in the formulation is in the range of from 240 mg/ml to 260 mg/ml.

In a most preferred embodiment the concentration of ethanol in the pharmaceutical formulation is 250 mg/ml.

In preferred embodiments, the concentration of ethanol in the formulation is in the range of from 1 % to 50 % (v/v). Preferably the concentration of ethanol in the formulation is in the range of from 5 % to 50 % (v/v). More preferably, the concentration of ethanol in the formulation is in the range of from 10 % to 40 % (v/v), more preferably 20 % to 40 % (v/v). In a further preferred embodiment, the concentration of ethanol in the formulation is in the range of from 25 % to 35 % (v/v). In certain preferred embodiments the concentration of ethanol in the formulation is 31% (v/v).

In preferred embodiments, the concentration of ethanol in the formulation is in the range of from 10 mg/ml to 1000 mg/ml. Preferably the concentration of ethanol in the formulation is in the range of from 50 mg/ml to 500 mg/ml. More preferably, the concentration of ethanol in the formulation is in the range of from 100 mg/ml to 400 mg/ml, more preferably 200 mg/ml to 300 mg/ml. In a further preferred embodiment, the concentration of ethanol in the formulation is in the range of from 240 mg/ml to 250 mg/ml.

In a most preferred embodiment the concentration of ethanol in the pharmaceutical formulation is 242 mg/ml.

In preferred embodiments, the concentration of ethanol in the formulation is in the range of from 1 % to 50 % (v/v). Preferably the concentration of ethanol in the formulation is in the range of from 5 % to 50 % (v/v). More preferably, the concentration of ethanol in the formulation is in the range of from 10 % to 40 % (v/v), more preferably 20 % to 40 % (v/v). In a further preferred embodiment, the concentration of ethanol in the formulation is in the range of from 25 % to 35 % (v/v). In certain preferred embodiments the concentration of ethanol in the formulation is 30% (v/v). In certain preferred embodiments the concentration of ethanol in the formulation is 33% (v/v).

The pharmaceutical formulation of the invention comprises benzyl alcohol (also known as benzenemethanol).

In preferred embodiments, the concentration of benzyl alcohol in the formulation is in the range of from 1 mg/ml to 100 mg/ml. Preferably the concentration of benzyl alcohol in the formulation is in the range of from 5 mg/ml to 50 mg/ml. More preferably, the concentration of benzyl alcohol in the formulation is in the range of from 10 mg/ml to 40 mg/ml, more preferably 20 mg/ml to 40 mg/ml. In a further preferred embodiment, the concentration of benzyl alcohol in the formulation is in the range of from 30 mg/ml to 35 mg/ml.

In a most preferred embodiment the concentration of benzyl alcohol in the pharmaceutical formulation is 31 mg/ml.

In preferred embodiments, the concentration of benzyl alcohol in the formulation is in the range of from 0.1 % to 10% (v/v). Preferably the concentration of benzyl alcohol in the formulation is in the range of from 0.5 % to 10 % (v/v).

Preferably, the concentration of benzyl alcohol in the formulation is in the range of from 1 % to 4 % (v/v), more preferably 2 % to 4 % (v/v). In a further preferred embodiment, the concentration of benzyl alcohol in the formulation is in the range of from 2.5 % to 3.5 % (v/v). In a further preferred embodiment, the concentration of benzyl alcohol in the formulation is in the range of from 2.8 % to 3.3 % (v/v). In certain preferred embodiments the concentration of benzyl alcohol in the formulation is 3% (v/v).

Alternatively and preferably, the concentration of benzyl alcohol in the formulation is in the range of from 4 % to 10 % (v/v), more preferably 4 % to 8 % (v/v). In a further preferred embodiment, the concentration of benzyl alcohol in the formulation is in the range of from 5 % to 7% (v/v). In a further preferred embodiment, the concentration of benzyl alcohol in the formulation is in the range of from 5.5 % to 6.5 % (v/v). In certain preferred embodiments the concentration of benzyl alcohol in the formulation is 6% (v/v).

In certain embodiments, the formulation has a pH in the range of from pH 3 to 4, preferably from pH 3.5 to 4.0. In certain embodiments, the formulation has a pH of 3.7.

In certain embodiments, the formulation of the invention has a pH in the range of from pH 5 to 8. In certain preferred embodiments, the pharmaceutical formulation has a pH in the range of from pH 5.0 to 7.8.

Preferably, the pharmaceutical formulation has a pH in the range of from pH 7.3 to 7.8.

Alternatively and preferably, the pharmaceutical formulation has a pH in the range of from pH 5.0 to 7.3, preferably a pH in the range of pH 5.0-7.0.

Preferably the pharmaceutical formulation has a pH in the range of from pH 6.0 to 7.0

Alternatively and preferably, the pharmaceutical formulation has a pH in the range of from pH 5.0 to 6.0.

The pH of the formulation can be adjusted by addition of a suitable organic acid, for example citric acid, acetic acid, tartaric acid, malic acid or succinic acid, preferably citric acid.

In certain preferred embodiments the formulation further comprises citric acid.

In a preferred embodiment the formulation comprises citric acid and has a pH of less than 7.3, for example a pH in the range of pH 5.0 to 7.3, preferably a pH in the range of pH 5.0-7.0. Preferably the formulation comprises citric acid and has a pH in the range of from 6.0-7.0. Alternatively and preferably, the formulation comprises citric acid and has a pH in the range of from 5.0-6.0.

In alternative preferred embodiments the formulation does not include citric acid.

In certain preferred embodiments the pharmaceutical formulation comprises:
50 mg/ml Etoposide toniribate;
750 mg/ml Polysorbate 80; and
250 mg/ml physiological acceptable organic solvent, such as ethanol.

In certain preferred embodiments, the pharmaceutical formulation comprises:
91 mg/ml Etoposide toniribate;
631 mg/ml Polysorbate 80; and
252 mg/ml physiological acceptable organic solvent, such as ethanol.

In certain preferred embodiments the pharmaceutical formulation consists essentially of:
50 mg/ml Etoposide toniribate;
750 mg/ml Polysorbate 80; and
250 mg/ml physiological acceptable organic solvent, such as ethanol.

In certain preferred embodiments the pharmaceutical formulation consists essentially of:
91 mg/ml Etoposide toniribate;
631 mg/ml Polysorbate 80; and
252 mg/ml physiological acceptable organic solvent, such as ethanol.

In such embodiments, the formulation may further comprise a suitable acid (e.g. citric acid) such that the formulation has the desired pH, as described above.

In certain preferred embodiments the pharmaceutical formulation consists of:
50 mg/ml Etoposide toniribate;
750 mg/ml Polysorbate 80; and
250 mg/ml physiological acceptable organic solvent, such as ethanol.

In certain preferred embodiments the pharmaceutical formulation consists of:
91 mg/ml Etoposide toniribate;
631 mg/ml Polysorbate 80; and
252 mg/ml physiological acceptable organic solvent, such as ethanol.

In a preferred aspect is provided a pharmaceutical product comprising a vial filled with 4ml of a pharmaceutical formulation of the invention as described herein. In preferred such embodiments, the pharmaceutical formulation according to the invention comprises:
0.2g of etoposide toniribate,
3g of polysorbate 80, and
1g ethanol.

In certain preferred embodiments the pharmaceutical formulation comprises:
10 mg/ml Etoposide toniribate;
650 mg/ml PEG 300;
80 mg/ml Polysorbate 80;
242 mg/ml Ethanol; and
31 mg/ml Benzyl alcohol.

In certain preferred embodiments, the pharmaceutical formulation comprises:
20 mg/ml Etoposide toniribate;
650 mg/ml PEG 300;
80 mg/ml Polysorbate 80;
242 mg/ml Ethanol; and
31 mg/ml Benzyl alcohol.

In such embodiments, the formulation may further comprise a suitable acid (e.g. citric acid) such that the formulation has the desired pH, as described above.

In certain preferred embodiments the pharmaceutical formulation consists essentially of:
10 mg/ml Etoposide toniribate;
650 mg/ml PEG 300;
80 mg/ml Polysorbate 80;
242 mg/ml Ethanol; and
31 mg/ml Benzyl alcohol.

In certain preferred embodiments the pharmaceutical formulation consists essentially of:
20 mg/ml Etoposide toniribate;
650 mg/ml PEG 300;
80 mg/ml Polysorbate 80;
242 mg/ml Ethanol; and
31 mg/ml Benzyl alcohol.

In such embodiments, the formulation may further comprise a suitable acid (e.g. citric acid) such that the formulation has the desired pH, as described above.

In certain preferred embodiments the pharmaceutical formulation consists of:
10 mg/ml Etoposide toniribate;
650 mg/ml PEG 300;
80 mg/ml Polysorbate 80;
242 mg/ml Ethanol; and
31 mg/ml Benzyl alcohol.

In certain preferred embodiments the pharmaceutical formulation consists of:
20 mg/ml Etoposide toniribate;
650 mg/ml PEG 300;
80 mg/ml Polysorbate 80;
242 mg/ml Ethanol; and
31 mg/ml Benzyl alcohol.

The formulations of the present invention are particularly advantageous due to their stability, as demonstrated in the accompanying Examples. As used herein, a stable pharmaceutical formulation is one in which the etoposide toniribate retains its physical stability and/or chemical stability and/or biological activity upon storage at the intended storage temperature (e.g. 2-8°C or room temperature). For example, a stable pharmaceutical formulation, between the time that it is made and the time that it is used (or reaches the end of its intended shelf-life), does not undergo any changes in its physical, chemical or biological properties which renders it unsafe or ineffective for its intended pharmaceutical use.

Stability of etoposide toniribate formulations can be determined by, for example, assessing physical stability by the level of particulates accumulated in the formulation as a result of precipitation.

In preferred embodiments, formulations according to the invention exhibit no precipitation after storage at 2-8°C for 72 hours.

Stability may also be assessed by measurement of the change in percentage of impurities in the formulation over time. Impurity levels can be determined by methods known to the person skilled in the art, for example chromatography methods.

In certain embodiments, formulations of the invention exhibit an increase in percentage of total impurities in the formulation of less than 0.5% over 3 months at 2-8°C, preferably an increase of total impurities in the formulation of less than 0.4% over 3 months at 2-8°C. In certain embodiments, formulations of the invention exhibit an increase in percentage of total impurities in the formulation of less than 0.5% over 5 months at 2-8°C, preferably an increase of total impurities in the formulation of less than 0.4% over 5 months at 2-8°C. In certain preferred embodiments, formulations of the invention exhibit an increase in percentage of total impurities in the formulation of less than 0.3% over 5 months at 2-8°C, preferably an increase of total impurities in the formulation of less than 0.2% over 5 months at 2-8°C. (Changes in percentage of impurity are given in absolute terms - i.e. an increase of total impurities from 0.1% to 0.3% over the given period is a change of 0.2%.)

In certain embodiments, formulations of the invention exhibit an increase in percentage of the cis-Cap7.1 impurity in the formulation of less than 0.3% over 3 months at 2-8°C. In certain embodiments, formulations of the invention exhibit an increase in percentage of the cis-Cap7.1 impurity in the formulation of less than 0.3% over 5 months at 2-8°C, preferably less than 0.2% over 5 months at 2-8°C.

Stability may also be assessed by measurement of the changes in etoposide toniribate concentration over time.

In certain preferred embodiments, formulations according to the invention exhibit a decrease in concentration of etoposide toniribate of no more than 0.5 mg/ml over 5 months at 2-8°C. In certain preferred embodiments, formulations according to the invention exhibit a decrease in concentration of etoposide toniribate of no more than 5% of the starting concentration over 5 months at 2-8°C.

Stability may also be determined by assessing chemical stability, indicated by the level of purity of the API over time. Purity levels can be determined by methods known to the person skilled in the art, for example chromatography methods, preferably HPLC.

In preferred embodiments, formulations according to the invention exhibit no visible precipitation after storage at 2-8°C for up to 72 hours, preferably up to 7 days. Preferably, formulations according to the invention exhibit no visible precipitation after storage at 2-8°C for up to 12 days, optionally up to 19 days, up to 26 days, up to 39 days, up to 49 days. Preferably, formulations according to the invention exhibit no visible precipitation after storage at 2-8°C for up to 12 weeks.

In preferred embodiments, formulations according to the invention exhibit no visible precipitation after storage at 20-25°C for up to 72 hours, preferably up to 7 days. Preferably, formulations according to the invention exhibit no visible precipitation after storage at 20-25°C for up to 12 days, optionally up to 14 days, optionally up to 19 days, up to 24 days, up to 26 days, up to 28 days, up to 35 days, up to 39 days, up to 42 days, up to 49 days, up to 56 days, up to 63 days, optionally up to 75 days. Preferably, formulations according to the invention exhibit no visible precipitation after storage at 20-25°C for up to 12 weeks. Preferably, formulations according to the invention exhibit no visible precipitation after storage at 20-25°C for up to 90 days. In such embodiments, storage at 20-25°C is at 60% relative humidity (RH).

In preferred embodiments, formulations according to the invention exhibit no visible precipitation after storage at 30°C for up to 7 days. Preferably, formulations according to the invention exhibit no visible precipitation after storage at 30°C for up to 14 days, optionally up to 24 days, up to 28 days, up to 35 days, up to 42 days, up to 49 days, up to 56 days, up to 63 days, optionally up to 75 days. Preferably, formulations according to the invention exhibit no visible precipitation after storage at 30°C for up to 12 weeks. Preferably, formulations according to the invention exhibit no visible precipitation after storage at 30°C for up to 90 days. In such embodiments, storage at 30°C is at 65% relative humidity (RH).

In preferred embodiments, formulations according to the invention exhibit no visible precipitation after storage at 40°C for up to 7 days. Preferably, formulations according to the invention exhibit no visible precipitation after storage at 30°C for up to 14 days, optionally up to 24 days, up to 28 days, up to 35 days, up to 42 days, up to 49 days, up to 56 days, up to 63 days, optionally up to 75 days. Preferably, formulations according to the invention exhibit no visible precipitation after storage at 40°C for up to 12 weeks. Preferably, formulations according to the invention exhibit no visible precipitation after storage at 40°C for up to 90 days. In such embodiments, storage at 40°C is at 75% relative humidity (RH).

In preferred embodiments, formulations according to the invention exhibit greater than 99% API purity after storage at 2-8°C for up to 72 hours, preferably up to 7 days. Preferably, formulations according to the invention exhibit greater than 99% API purity after storage at 2-8°C for up to 12 days, optionally up to 19 days, up to 26 days, up to 39 days, up to 49 days. Preferably, formulations according to the invention exhibit greater than 99% API purity after storage at 2-8°C for up to 12 weeks.

In preferred embodiments, formulations according to the invention exhibit greater than 99% API purity after storage at 20-25°C for up to 72 hours, preferably up to 7 days. Preferably, formulations according to the invention exhibit greater than 99% API purity after storage at 20-25°C for up to 12 days, optionally up to 14 days, optionally up to 19 days, up to 24 days, up to 26 days, up to 28 days, up to 35 days, up to 39 days, up to 42 days, up to 49 days, up to 56 days, up to 63 days, optionally up to 75 days. Preferably, formulations according to the invention exhibit greater than 99% API purity after storage at 20-25°C for up to 12 weeks. Preferably, formulations according to the invention exhibit greater than 99% API purity after storage at 20-25°C for up to 90 days. In such embodiments, storage at 20-25°C is at 60% relative humidity (RH).

In preferred embodiments, formulations according to the invention exhibit greater than 99% API purity after storage at 30°C for up to 7 days. Preferably, formulations according to the invention exhibit greater than 99% API purity after storage at 30°C for up to 14 days, optionally up to 24 days, up to 28 days, up to 35 days, up to 42 days, up to 49 days, up to 56 days, up to 63 days, optionally up to 75 days. Preferably, formulations according to the invention exhibit greater than 99% API purity after storage at 30°C for up to 12 weeks. Preferably, formulations according to the invention exhibit greater than 99% API purity after storage at 30°C for up to 90 days. In such embodiments, storage at 30°C is at 65% relative humidity (RH).

In preferred embodiments, formulations according to the invention exhibit greater than 99% API purity after storage at 40°C for up to 7 days. Preferably, formulations according to the invention exhibit greater than 99% API purity after storage at 30°C for up to 14 days, optionally up to 24 days, up to 28 days, up to 35 days, up to 42 days, up to 49 days, up to 56 days, up to 63 days, optionally up to 75 days. Preferably, formulations according to the invention exhibit greater than 99% API purity after storage at 40°C for up to 12 weeks. Preferably, formulations according to the invention exhibit greater than 99% API purity after storage at 40°C for up to 90 days. In such embodiments, storage at 40°C is at 75% relative humidity (RH).

In a further aspect the invention provides a method of making an etoposide toniribate formulation according to the invention, the method comprising: (a) mixing the polysorbate and ethanol, and stirring the mixture; (b) adding the etoposide toniribate to the mixture obtained in step (a) and stirring the resultant mixture. In a further preferred embodiment of the this aspect, the method is for making an oral etoposide toniribate formulation, the method comprising: (a) mixing the polysorbate and ethanol, and stirring the mixture; (b) adding the etoposide toniribate to the mixture obtained in step (a) and stirring the resultant mixture, and a further step (x) formulating the composition in an oral dosage form, such as preferably a gel, pill, tablet, caplet, hard capsule and/or soft capsule.

In a further aspect the invention provides a method of making, preferably an oral composition, of etoposide toniribate formulation according to the invention, the method comprising: (a) mixing the etoposide toniribate, ethanol, and benzyl alcohol and stirring the mixture; (b) adding the PEG 300 and the polysorbate to the mixture obtained in step (a) and stirring to obtain a solution. In certain embodiments the rate of stirring is increased from step (a) to step (b).

In certain embodiments the method further comprises adjusting the pH of the mixture obtained in step (b) through addition of an acid (e.g. citric acid).

In certain embodiments the method further comprises sterile filtering the etoposide toniribate solution obtained in step (b). In certain preferred embodiments, the polysorbate is polysorbate 80 having a pH of less than 7, preferably in the range of from pH 3-7.

The invention in another aspect provides a lyophilized composition of etoposide toniribate, which is stable at temperatures higher than -50°C.

In another aspect, the present invention provides a method for producing a lyophilized composition of etoposide toniribate, wherein the method comprises the following steps:
a. providing a liquid comprising at least etoposide toniribate and a cyclodextrin;
b. optionally filtrating the liquid;
c. cooling the liquid to a freezing temperature, wherein the cooling is performed at a defined cooling rate;
d. freezing the liquid at the freezing temperature in order to obtain a frozen liquid; and
e. drying the frozen liquid obtained in step d) in order to obtain a lyophilized composition comprising etoposide toniribate.

In a preferred embodiment, the liquid in step a. is buffered at a pH between about 4 and about 7, preferably between about 5,5 and about 6,5.

In a preferred embodiment of the invention the liquid in step a. is a buffered solution comprising a salt, preferably 4-(2-hydroxyethyl)-1-piperazineethanesulfonic acid (HEPES).

The term "cyclodextrin" as disclosed herein should be interpreted broadly to include the natural cyclodextrins and their derivatives, including the alkylated and hydroxyalkylated derivatives and the branched cyclodextrins. Cyclodextrins and their derivatives which have been previously described as useful for complexation with drugs are of particular interest herein. In addition to α-, β- and γ-cyclodextrin, the ether and mixed ether derivatives and those derivatives bearing sugar residues are of special interest. Especially useful herein are the hydroxyethyl, hydroxypropyl (including 2- and 3-hydroxypropyl) and dihydroxypropyl ethers, their corresponding mixed ethers and further mixed . ethers with methyl or ethyl groups, such as methyl-hydroxyethyl, ethyl- hydroxyethyl and ethyl-hydroxypropyl ethers of α-, β- and γ-cyclodextrin. Hydroxypropyl-β-cyclodextrin and its preparation by propylene oxide addition to β-cyclodextrin, and hydroxyethyl-β-cyclodextrin and its preparation by ethylene oxide addition to β-cyclodextrin, were described in a patent of Gramera et al. (U.S. Pat. No. 3,459,731, issued August 1969) over 20 years ago. Other useful cyclodextrin derivatives are maltosyl, glucosyl and maltotriosyl derivatives of β-and γ-cyclodextrin, which may contain one or more sugar residues, e.g. glucosyl or diglucosyl, maltosyl or dimaltosyl, as well as various mixtures thereof, e.g. a mixture of maltosyl and dimaltosyl derivatives. Other useful cyclodextrin derivatives comprise anionic functional groups such as sulfobutylether derivatives, sulfonates, phosphates, and the like. Specific examples of cyclodextrin derivatives for use herein include hydroxypropyl-β- cyclodextrin, hydroxypropyl-γ-cyclodextrin, sulfobutylether-β-cyclodextrin, and sulfobutylether-γ-cyclodextrin, as well as hydroxyethyl-β-cyclodextrin, hydroxyethyl-γ-cyclodextrin, dihydroxypropyl-β-cyclodextrin, glucosyl-β- cyclodextrin, diglucosyl-β-cyclodextrin, maltosyl-β-cyclodextrin, maltosyl-γ- cyclodextrin, maltotriosyl-β-cyclodextrin, maltotriosyl-γ-cyclodextrin and dimaltosyl-β-cyclodextrin, and mixtures thereof such as maltosyl-β-cyclodextrin/dimaltosyl-β-cyclodextrin. Procedures for preparing such cyclodextrin derivatives are well-known, for example, from Bodor U.S. Pat. No. 5,024,998, dated Jun. 18, 1991, expressly incorporated herein by reference, and references cited therein

In a preferred embodiment a cyclodextrin is beta-cyclodextrin, such as β-Cyclodextrin Sulfobutyl ether (Captisol^{®}), Hydroxypropyl-β-Cyclodextrin (Cavitron^{®}).

In a further aspect of the invention the formulation is a lyophilized composition of etoposide toniribate, preferably wherein the formulation is comprising etoposide toniribate and a cyclodextrin, optionally a salt.

A lyophilized composition of etoposide toniribate is preferably obtainable by a method as disclosed herein, for example in the appended examples.

In a further aspect the invention provides a kit comprising a pharmaceutical formulation according to the invention. In certain embodiments the kit further comprises instructions for the use of the formulation. In certain embodiments the pharmaceutical formulation is provided in the kit in package containing oral dosage forms, such as a gel, pill, tablet, caplet, hard capsule or soft capsule.

In certain embodiments the pharmaceutical formulation is provided in a vial, and preferably is at a fill volume in the range of from 5 ml to 20 ml. In certain preferred embodiments the pharmaceutical formulation is provided in a vial at a fill volume of 10 ml.

In preferred embodiments the vial is a photo-protective vial, for example an amber vial. In certain embodiments the pharmaceutical formulation is provided in a vial at a fill volume in the range of from 1 ml to 20 ml. In certain preferred embodiments the pharmaceutical formulation is provided in a vial at a fill volume in the range of from 4 ml to 10 ml. In certain preferred embodiments the pharmaceutical formulation is provided in a vial at a fill volume in the range of from 4 ml to 6 ml.

In certain embodiments the kit comprises instructions for preparing an infusion solution according to the invention. In certain embodiments the kit comprises instructions for treating a patient in accordance with the methods of therapy provided herein.

In certain embodiments, the kit further comprises a diluent. In certain such embodiments the diluent is selected from sodium chloride solution (optionally a 0.45% or 0.9% sodium chloride solution), glucose solution (optionally 5% glucose solution), and water for injection.

Intravenous administration of etoposide toniribate to a patient is preferred. To prepare an infusion solution suitable for effective intravenous administration, the pharmaceutical formulations of the invention need to be diluted in a suitable diluent.

As demonstrated herein, the infusion solutions prepared from by diluting the pharmaceutical formulations of the invention have the advantage that they are stable at room temperature (controlled at 20-25°C, 60% RH). As shown in the examples, infusion solutions prepared using any of glucose solution, sodium chloride solution (saline) or water for injection (WFI) exhibited no precipitation when stored for 24 hours at room temperature (20- 25°C, 60% relative humidity (RH).

As shown in the examples, infusion solutions prepared using any of glucose solution, sodium chloride solution (0.45% or 0.9%) or water for injection (WFI) exhibited physical stability (as shown by lack of precipitation) and chemical stability (>99% purity) when stored for 24 hours at room temperature. This stability makes the infusion solutions convenient to prepare and handle prior to administration, making the infusion solutions particularly advantageous.

Accordingly, in a further aspect the invention provides an infusion solution comprising a pharmaceutical formulation according to the invention and a diluent. The invention also provides a method of preparing an infusion solution, the method comprising diluting a pharmaceutical formulation according to the invention in a diluent. The invention further provides an infusion solution prepared according to such a method.

In certain embodiments of each of these aspects the diluent is selected from sodium chloride solution (optionally 0.45% sodium chloride solution or 0.9% sodium chloride solution), glucose solution (optionally 5% glucose solution), and water for injection. In certain embodiments the diluent is sodium chloride (saline), optionally 0.45% or 0.9% saline. In certain embodiments the diluent is glucose solution, optionally 5% glucose solution. In certain embodiments the diluent is water for injection (WFI). Using WFI as the diluent is particularly preferred due to the beneficial osmolality of the resultant infusion solution being closest to the physiological osmolality of 280-290 mOsm/kg, as shown in the Examples.

In certain embodiments, the etoposide toniribate concentration in the infusion solution is in the range of from 0.1 mg/ml to 5 mg/ml. In certain preferred embodiments, the etoposide toniribate concentration in the infusion solution is in the range of from 0.5 mg/ml to 3 mg/ml.

In certain preferred embodiments, the etoposide toniribate concentration in the infusion solution is in the range of from 0.5 mg/ml to 0.8 mg/ml.

In certain preferred embodiments, the etoposide toniribate concentration in the infusion solution is 0.5 mg/ml or 0.8 mg/ml. In certain alternative preferred embodiments, the etoposide toniribate concentration in the infusion solution is 0.7 mg/ml or 2.9 mg/ml.

In certain embodiments, the etoposide toniribate concentration in the infusion solution is in the range of from 1 mg/ml to 10 mg/ml. In certain preferred embodiments, the etoposide toniribate concentration in the infusion solution is in the range of from 1 mg/ml to 5 mg/ml, preferably 2 mg/ml to 4 mg/ml. In certain preferred embodiments, the etoposide toniribate concentration in the infusion solution is in the range of from 3 mg/ml to 4 mg/ml, preferably 3.0 mg/ml to 3.5 mg/ml. In certain preferred embodiments, the etoposide toniribate concentration in the infusion solution is 3.1 mg/ml. In certain preferred embodiments, the etoposide toniribate concentration in the infusion solution is 3.2 mg/ml.

The skilled person will appreciate that the concentration of the formulation components in infusion solutions according to the invention reflects the dilution of etoposide toniribate relative to the pharmaceutical formulation from which the solution is prepared. For example, where an infusion solution having a concentration of etoposide toniribate of 3 mg/ml is prepared from a pharmaceutical formulation having an etoposide toniribate concentration of 30 mg/ml, the concentration of the other components in the infusion solution will be 10x more dilute versus the pharmaceutical formulation concentrations.

In a further aspect the invention provides a method of treating cancer in a patient in need thereof, the method comprising administering to the patient an effective amount of a pharmaceutical formulation according to the invention or an infusion solution according to the invention.

In a preferred embodiment, the method comprises a step of orally administering a composition of etoposide toniribate formulation as described herein.

In other certain preferred embodiments, the formulation or infusion solution is administered intravenously.

In some embodiments, the method comprises a step of resuspending a lyophilized etoposide toniribate composition of the invention, and a subsequent administration of the resuspended composition, for example intravenously.

In certain preferred embodiments, the cancer is selected from the group consisting of biliary tract cancer, adenocarcinoma (e.g. colon adenocarcinoma and colorectal adenocarcinoma), hypopharynx cancer (e.g. squamous cell hypopharynx cancer, especially with pulmonary metastases), lung cancer (e.g. lung carcinoma, small cell lung cancer and squamous cell cancer of the lung), diffuse large cell lymphoma, Burkitt's lymphoma, Hodgkin lymphoma, Non-Hodgkin lymphoma, histiocytic lymphoma, lymphatic lymphoma, acute T-cell leukaemia, pre-B-acute lymphoblastic leukaemia, thymus carcinoma (e.g. sarcomatoid thymus carcinoma, especially with pulmonary metastases), urothelium carcinoma, testicular cancer (e.g. testicular germ cell tumour, seminoma and non-seminoma, especially with renal, pulmonary, retroperitoneal, hepatic and/or cerebral metastases), prostate cancer, bladder cancer, AIDS-related Kaposi's sarcoma, Ewing sarcoma, rhabdomyosarcoma, neuroblastoma (in particular paediatric neuroblastoma, especially advanced paediatric neuroblastoma), ovarian cancer (e.g. ovarian germ cell tumour, or ovarian carcinoma), and breast cancer. In preferred embodiments the method is a method of treating biliary tract cancer.

As used herein, "patient" refers to a subject to be administered therapy, for example for cancer. In preferred embodiments of all aspects of the invention, the patient is a human patient.

As used in the claims, "comprising" is given its conventional construction of meaning that any further component, step or feature can be present in addition to those recited in the claim. "Consisting essentially of" is intended to mean further components may be present provided they do not materially affect the essential characteristics of the formulation. "Consisting of" is given its convention construction of meaning no further component, step or feature is present beyond those recited in the claim.

With the above context, the following consecutively itemized A embodiments provide further specific itemized embodiments and aspects of the invention, which shall be understood and read in context with the above detailed description of the invention:
Item 1: A liquid or solid pharmaceutical formulation comprising:
   etoposide toniribate;
   a polysorbate; and
   optionally, physiological acceptable organic solvent, such as Ethanol.
Item 2: The liquid or solid pharmaceutical formulation of item 1, the formulation is an oral dosage form, such as a pill, tablet, caplet, hard capsule or soft capsule.
Item 3: The liquid or solid pharmaceutical formulation of item 1 or 2, wherein the pharmaceutical formulation is in powder form, such as a lyophilized (dried) powder.
Item 4: The liquid or solid pharmaceutical formulation of any one of items 1 to 3, comprising etoposide toniribate at a concentration in the range of from 50 mg/ml to 100 mg/ml.
Item 5: The liquid or solid pharmaceutical formulation of any one of items 1 to 4, wherein the polysorbate is polysorbate 80.
Item 6: The liquid or solid pharmaceutical formulation of any preceding item wherein the polysorbate concentration is in the range of from 600 mg/ml to 800 mg/ml.
Item 7: The liquid or solid pharmaceutical formulation of any preceding item wherein the polysorbate concentration is 750 mg/ml.
Item 8: The liquid or solid pharmaceutical formulation of any preceding item wherein the ethanol concentration is in the range of from 200 mg/ml to 300 mg/ml.
Item 9: The liquid or solid pharmaceutical formulation of any preceding item wherein the concentration of physiological acceptable organic solvent, such as Ethanol, is 250 mg/ml.
Item 10: The liquid or solid pharmaceutical formulation of any preceding item, wherein the liquid or solid pharmaceutical formulation comprises:
   50 mg/ml Etoposide toniribate;
   750 mg/ml Polysorbate 80; and
   250 mg/ml physiological acceptable organic solvent, such as Ethanol.
Item 11: The liquid or solid pharmaceutical formulation of any one of items 1-7, wherein the liquid or solid pharmaceutical formulation comprises:
   91 mg/ml Etoposide toniribate;
   631 mg/ml Polysorbate 80; and
   252 mg/ml Ethanol.
Item 12: The liquid or solid pharmaceutical formulation of any one of items 1-7, wherein the liquid or solid pharmaceutical formulation consists, or consists essentially of:
   50 mg/ml Etoposide toniribate;
   750 mg/ml Polysorbate 80; and
   250 mg/ml Ethanol.
Item 13: The liquid or solid pharmaceutical formulation of any one of items 1-7, wherein the liquid or solid pharmaceutical formulation consists, or consists essentially of:
   91 mg/ml Etoposide toniribate;
   631 mg/ml Polysorbate 80; and
   252 mg/ml Ethanol.
Item 14: The liquid or solid pharmaceutical formulation of any preceding item, wherein the liquid or solid pharmaceutical formulation has a pH in the range of from pH 3 to 4.
Item 15: The liquid or solid pharmaceutical formulation of any preceding item, wherein the liquid or solid pharmaceutical formulation has a pH of 3.7.
Item 16: A method of preparing an infusion solution comprising diluting the liquid pharmaceutical formulation according to any one of items 1-15 in a diluent; or dissolving a solid pharmaceutical formulation according to any one of items 1-15 in a diluent.
Item 17: An infusion solution prepared by diluting a pharmaceutical formulation according to any of items 1-13 in a diluent.
Item 18: An infusion solution comprising the liquid or solid pharmaceutical formulation according to any one of items 1-13 and a diluent.
Item 19: The method according to item 14 or the infusion solution according to item 15 or 16, wherein the diluent is selected from: water for injection; 5% glucose solution; 0.45% NaCl saline, and 0.9% NaCl saline.
Item 20: The method or an infusion solution according to item 17, wherein the diluent is water for injection.
Item 21: The method or the infusion solution according to any one of items 14-18, wherein the etoposide toniribate concentration in the infusion solution is in the range of from 1 mg/ml to 10 mg/ml.
Item 22: The method or the infusion solution according to any one of items 14-19, wherein the etoposide toniribate concentration in the infusion solution is in the range of from 1 mg/ml to 5 mg/ml.
Item 23: The method or the infusion solution according to any one of items 14-20, wherein the etoposide toniribate concentration in the infusion solution is 3.1 mg/ml.
Item 24: A kit comprising: the liquid or solid pharmaceutical formulation of any one of items 1-13; and a diluent.
Item 25: The kit of item 22, wherein the diluent is selected from: water for injection; 5% glucose solution; 0.45% NaCl saline, and 0.9% NaCl saline.
Item 26: The liquid or solid pharmaceutical formulation according to any one of items 1-13 or the infusion solution according to any one of items 15-21, for use in therapy.
Item 27: The liquid or solid pharmaceutical formulation according to any one of items 1-13 or the infusion solution according to any one of items 15-21, for use in a method of treating cancer.
Item 28: The liquid or solid pharmaceutical formulation according to any one of items 1-13 or the infusion solution according to any one of items 15-21, for use in a method of treating biliary tract cancer
Item 29: A method of treating cancer in a patient in need thereof, the method comprising administering to the patient an effective amount of the liquid or solid pharmaceutical formulation according to any one of items 1-13, or the infusion solution according to any one of items 15-21.
Item 30: A liquid or solid pharmaceutical formulation comprising:
   Etoposide toniribate;
   PEG;
   a polysorbate;
   Ethanol;
   Benzyl alcohol.
Item 31: The liquid or solid pharmaceutical formulation of item 30, wherein the formulation is an oral dosage form.
Item 32: The liquid or solid pharmaceutical formulation of item 30 or 31, wherein the pharmaceutical formulation is in powder form, such as a lyophilized (dried) powder.
Item 33: The liquid or solid pharmaceutical formulation of any one of items 30 to 32, comprising etoposide toniribate at a concentration in the range of from 10 mg/ml to 20 mg/ml, optionally at a concentration of 10 mg/ml or 20mg/ml.
Item 34: The liquid or solid pharmaceutical formulation of any one of items 30 to 33, wherein the polysorbate is polysorbate 80.
Item 35: The liquid or solid pharmaceutical formulation of any one of items 30 to 34, wherein the PEG is PEG 200-600.
Item 36: The liquid or solid pharmaceutical formulation of any one of items 30 to 35, wherein the PEG is PEG 200-400.
Item 37: The liquid or solid pharmaceutical formulation of any one of items 30 to 36, wherein the PEG is PEG 300.
Item 38: The liquid or solid pharmaceutical formulation of any one of items 30 to 37, wherein the liquid or solid pharmaceutical formulation has a pH in the range of from pH 5.0 to 7.8.
Item 39: The liquid or solid pharmaceutical formulation of any one of items 30-38, wherein the liquid or solid pharmaceutical formulation has a pH in the range of from 7.3 to 7.8.
Item 40: The liquid or solid pharmaceutical formulation of any one of items 30-39, wherein the liquid or solid pharmaceutical formulation has a pH in the range of from 6.0 to 7.0.
Item 41: The liquid or solid pharmaceutical formulation of any one of items 30-40, wherein the liquid or solid pharmaceutical formulation has a pH in the range of from 5.0 to 6.0.
Item 42: The liquid or solid pharmaceutical formulation of any one of items 30 to 41, wherein the liquid or solid pharmaceutical formulation further comprises citric acid.
Item 43: The liquid or solid pharmaceutical formulation of any one of items 30 to 42, wherein the liquid or solid pharmaceutical formulation comprises:
   10 mg/ml Etoposide toniribate;
   650 mg/ml PEG 300;
   80 mg/ml Polysorbate 80;
   242 mg/ml Ethanol; and
   31 mg/ml Benzyl alcohol.
Item 44: The liquid or solid pharmaceutical formulation of any one of items 30 to 43, wherein the liquid or solid pharmaceutical formulation comprises:
   20 mg/ml Etoposide toniribate;
   650 mg/ml PEG 300;
   80 mg/ml Polysorbate 80;
   242 mg/ml dehydrated ethanol; and
   31 mg/ml Benzyl alcohol.
Item 45: The liquid or solid pharmaceutical formulation of any one of items 30 to 44, wherein the liquid or solid pharmaceutical formulation consists of, or consists essentially of:
   10 mg/ml Etoposide toniribate;
   650 mg/ml PEG 300;
   80 mg/ml Polysorbate 80;
   242 mg/ml dehydrated ethanol; and
   31 mg/ml Benzyl alcohol.
Item 46: The liquid or solid pharmaceutical formulation of any one of items 30 to 45, wherein the liquid or solid pharmaceutical formulation consists of, or consists essentially of: 20 mg/ml Etoposide toniribate;
650 mg/ml PEG 300;
80 mg/ml Polysorbate 80;
242 mg/ml Ethanol; and
31 mg/ml Benzyl alcohol.
Item 47: A method of preparing an infusion solution comprising diluting the liquid or solid pharmaceutical formulation according to any one of items 30-46 in a diluent.
Item 48: An infusion solution prepared by diluting the liquid or solid pharmaceutical formulation according to any of items 30-46 in a diluent.
Item 49: An infusion solution comprising the liquid or solid pharmaceutical formulation according to any one of items 30-46 and a diluent.
Item 50: The method according to item 47 or the infusion solution according to item 48 or 49, wherein the diluent is selected from: water for injection; 5% glucose solution; and 0.9% NaCl saline.
Item 51: The method or the infusion solution according to item 50, wherein the diluent is water for injection.
Item 52: The method or the infusion solution according to any one of items 43-47, wherein the etoposide toniribate concentration in the infusion solution is in the range of from 0.1 mg/ml to 1 mg/ml.
Item 53: The method or the infusion solution according to any one of items 43-48, wherein the etoposide toniribate concentration in the infusion solution is in the range of from 0.5 mg/ml to 0.8 mg/ml.
Item 54: The method or the infusion solution according to any one of items 43-49, wherein the etoposide toniribate concentration in the infusion solution is 0.5 mg/ml or 0.8 mg/ml.
Item 55: A kit comprising: the liquid or solid pharmaceutical formulation of any one of items 28-42; and a diluent.
Item 56: The kit of item 51, wherein the diluent is selected from: water for injection; 5% glucose solution; and 0.9% NaCl saline.
Item 57: The liquid or solid pharmaceutical formulation according to any one of items 28-42, or an infusion solution according to any one of items 43-45, for use in therapy.
Item 58: The liquid or solid pharmaceutical formulation according to any one of items 28-42 or the infusion solution according to any one of items 43-50, for use in a method of treating cancer.
Item 59: The liquid or solid pharmaceutical formulation according to any one of items 28-42 or the infusion solution according to any one of items 43-50, for use in a method of treating biliary tract cancer
Item 60: A method of treating cancer in a patient in need thereof, the method comprising administering to the patient an effective amount of the liquid or solid pharmaceutical formulation according to any one of items 28-42, or the infusion solution according to any one of items 43-50.
Item 61: A method for producing a lyophilized composition of etoposide toniribate, wherein the method comprises the following steps:
   a. providing a liquid comprising at least etoposide toniribate and a cyclodextrin;
   b. optionally filtrating the liquid;
   c. cooling the liquid to a freezing temperature, wherein the cooling is performed at a defined cooling rate;
   d. freezing the liquid at the freezing temperature in order to obtain a frozen liquid; and
   e. drying the frozen liquid obtained in step d) in order to obtain a lyophilized composition comprising etoposide toniribate.
Item 62: The method of item 61, wherein the liquid in step a. is buffered at a pH between about 4 and about 7, preferably between about 5,5 and about 6,5.
Item 63: The method of item 61 or 62, wherein the liquid in step a. comprises a salt, preferably HEPES.
Item 64: The method of any one of items 61 to 63, wherein cyclodextrin is beta-cyclodextrin, such as β-Cyclodextrin Sulfobutyl ether (Captisol^{®}), Hydroxypropyl-β-Cyclodextrin (Cavitron^{®}).
Item 65: A lyophilized composition of etoposide toniribate, comprising etoposide toniribate and a cyclodextrin, optionally a salt.
Item 66: The lyophilized composition of etoposide toniribate of item 65, wherein the salt is HEPES.
Item 67: The lyophilized composition of etoposide toniribate of item 65 or 66, wherein the cyclodextrin is beta-cyclodextrin, such as β-Cyclodextrin Sulfobutyl ether (Captisol^{®}), Hydroxypropyl-β-Cyclodextrin (Cavitron^{®}).
Item 68: The lyophilized composition of etoposide toniribate of any one of items 65 to 67, obtainable by freeze-drying, preferably by a method of any one of items 61 to 64.
Item 69: A pharmaceutical package, comprising a lyophilized composition of etoposide toniribate according to any one of items 61 to 65.

### Examples

Etoposide toniribate as API was known to be poorly soluble in water, and unstable at acidic and basic pH. To date, the API has been formulated by dissolving it in Cremophor RH40 and ethanol. However, this formulation is cumbersome to use and lacks scalability. Experiments were undertaken to develop a new stable formulation of etoposide toniribate that is stable at refrigerator temperature (2-8°C) and compatible with acceptable infusion diluents, such that the infusion solution is stable for at least 2-3 hours at room temperature (assessed at controlled room temperature of 20-25°C, 60% relative humidity (RH)). It is also desirable for the infusion solution to have an osmolality close to blood osmolality (280-295 mOsm/Kg).

### Example 1

Formulation: 25 g Polysorbate 80 and 10 g Ethanol were mixed (polysorbate 80 having an acidic pH was used for all the exemplified formulations). To the resulting mixture of 36 ml 3.6 g CAP 7.1 were added with stirring. The solution was filtered with membrane 0.2pm and 1.1 ml filled into glass vials (5ml), giving 100mg of CAP7.1 per vial. The vials were sealed with 13mm bromobutyl rubber stoppers and Al-Crimp caps. The vials were stored at 5°C ± 3°C and 20-25°C/60% RH as well. The stability was examined by HPLC (method for related substances) over 12 weeks. The in-use stability was proofed in dilution with 0.9% NaCl solution. Results are compiled in table 1A (5°C ± 3°C), table 2A (20-25°C/60% RH) and table 3A (in-use) respectively.

The formulation was found to be stable for at least 12 weeks at 2-8°C and at 20-25°C with 60% relative humidity (Tables 1A and 2A). When an "in use" infusion solution was prepared to approximately 3.3 mg/ml in 0.9% saline, the infusion solution was stable for at least 6 hours at room temperature (20-25°C, 60% RH), with the 20 hour results also showing acceptable stability levels.

### Example 2

As the stability of the formulation based on polysorbate 80 and ethanol could be demonstrated in Example 1 a formulation containing 200 mg CAP 7.1 per vial has been tested. For better handling a bigger Vial (10R) was chosen and the filling volume was increased up to 4 ml (filled CAP7.1 concentration 50 mg/ml).

Formulation of example2

| | |
|---|---|
| One vial contains | 3g Polysorbate 80 |
| | 1g Ethanol |
| | 0.2 g CAP 7.1 |

### Filling volume 4 ml per vial

Primary packaging: 10R Vials HKI; Teflon coated rubber stoppers sealed with Al-crimp caps.

The vials were stored at 5°C ± 3°C; 20-25°C/60% RH; 30°C/65% RH and 40°C/75% RH as well. The stability was examined by HPLC (Method for related substances) over 90 days. The results are presented in Table 4A (API purity assay) and Table 5A (related substance HPLC).

Results: Based on the purity assay by area% of Table 4, the formulation is shown to be stable for at least 90 days at 5°C ± 3°C and at 20-25°C/60% RH. At 30°C/65% RH and 40°C/75% RH storage the assay dropped from 99.55% initial to 99.40% and 99.06% respectively. These values still indicate remarkable stability of the formulation.

The related substances data of Table 5A confirm this stability. The only "decomposition" product formed during storage at 30°C/65% RH and 40°C/75% RH was the compound at RRT 0.97. This is a known substance representing an isomer of CAP 7.1. Overall the formulation showed stability under all storage conditions for at least 90 days.

Repeat experiments across at least 4 batches produced across separate laboratories confirmed these results and further showed that the formulation was stable for at least 112 days at 5°C ± 3°C, at 20-25°C/60% RH and at 30°C/65% RH.

### Example 3

For administration to patients, the stable formulation needs to be diluted in an aqueous solution. To identify suitable diluents for the infusion solution, the formulation of Example 2 was diluted in various solutions to a concentration of approx. 3.1 mg/ml CAP7.1. and the stability and osmolality assessed. The diluents tested were isotonic (0.9%) sodium chloride solution, 0.45% sodium chloride solution, and 5% Glucose solution.

Infusion solutions were prepared as follows: One vial of the formulated concentrate, containing 200 mg of API, 3g Polysorbate 80 and 1g Ethanol was diluted with 60 ml diluent (water for injection, 0.9% NaCl solution, 0.45% NaCl solution or 5% Glucose solution). The resulting solution for infusion contains about 3.13 mg/ml of API.

Results: The osmolality of the different infusion solutions is shown below:

| **Diluent** | **Osmolality [mOsmol/kg]** |
|---|---|
| Water for injection | 360 |
| 0.45% NaCl solution | 526 |
| 0.9% NaCl solution | 689 |
| 5% Glucose solution | 719 |

The infusion solution prepared using water for injection shows an osmolality most-similar to blood osmolality (approx. 275-295 mOsm/kg). It is expected that an isotonic infusion solution could be prepared when about 70 ml WFI is used as diluent.

Stability of the infusion solutions is shown in Table 6A below. In summary, all the tested infusion solutions showed similar stability, each exhibiting chemical and physical stability when stored at room temperature for 24 hours. In particular, over24 hours for each infusion solution the content of CAP 7.1 decreased about 0.2%. The only decomposition product was Etoposide, which increased in the same range, indicative of hydrolytic decomposition. Physical stability measured by assessing visible and sub-visible particles was found to be highly stable, with very low levels of particles detected.

### Example 4

To assess photostability, a formulation was prepared according to Example 2, filled in amber glass vials and exposed to light providing an overall irradiated illumination of more than 1.2 million lux hours and an integrated near ultraviolet energy of nearly 200 watt hours/square metre. Additionally, one sample packed in secondary packaging (cardboard box) and one sample protected by wrapping in aluminium foil were also examined as dark controls. Comparative tabulated results of assay and chemical purity are provided in Tables 7A and 8A below.

Results: the data show that there was no change in assay or chemical purity following irradiation. It was demonstrated that the selected primary packaging itself (amber glass) is sufficient to provide light protection. Experiments testing photo-stability of the formulation under irradiation in transparent vials found that the formulation decomposed. Photo-protective primary packaging (e.g. amber vials) or secondary packaging (e.g. cardboard) should therefore be used for storage.

### Conclusions

Stability data from the batch formulations suggest a very stable medicinal product at refrigerated storage and also at 20-25°C/60% RH, as well as at higher temperatures. The infusion solutions prepared from the formulation meet all requirements for clinical use in terms of physical and chemical properties, in use stability of the infusion solutions, osmolality etc. Furthermore the formulation allows a comfortable use in hospital routine because of easy handling of the medicinal product during preparation of infusions and the in use stability of about 20 hours.

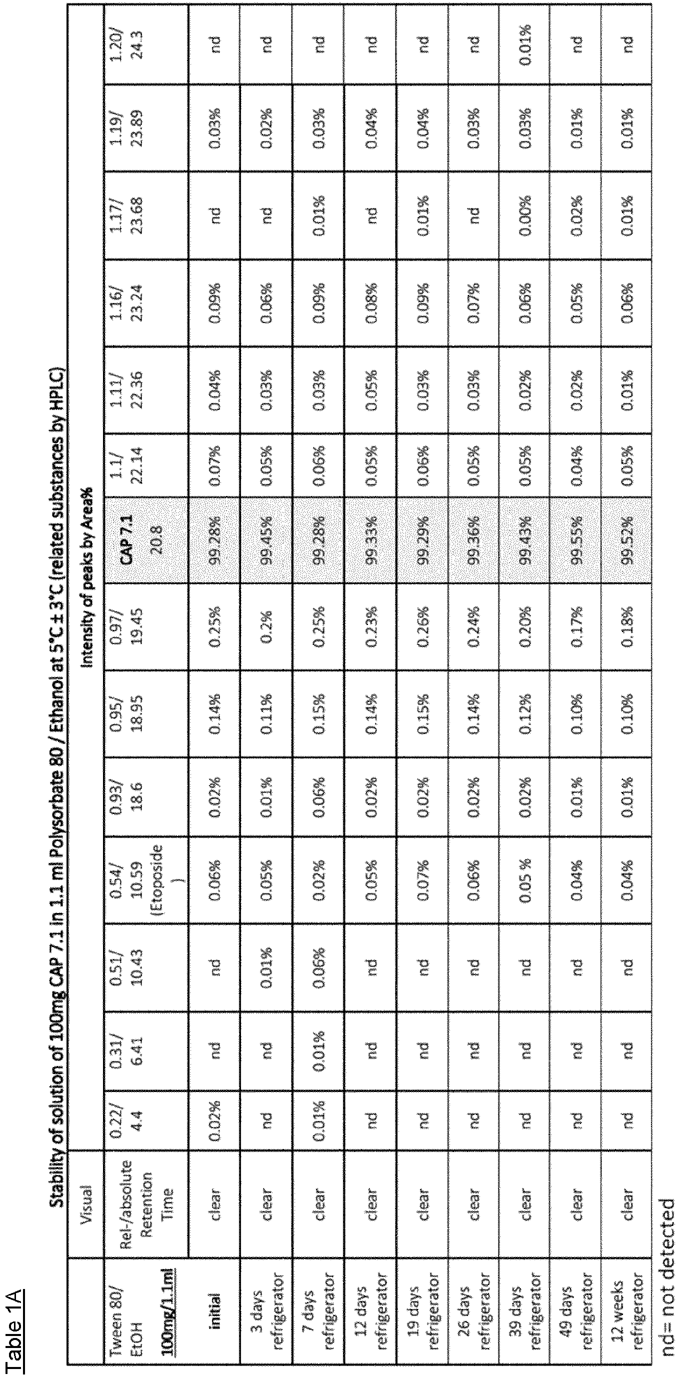

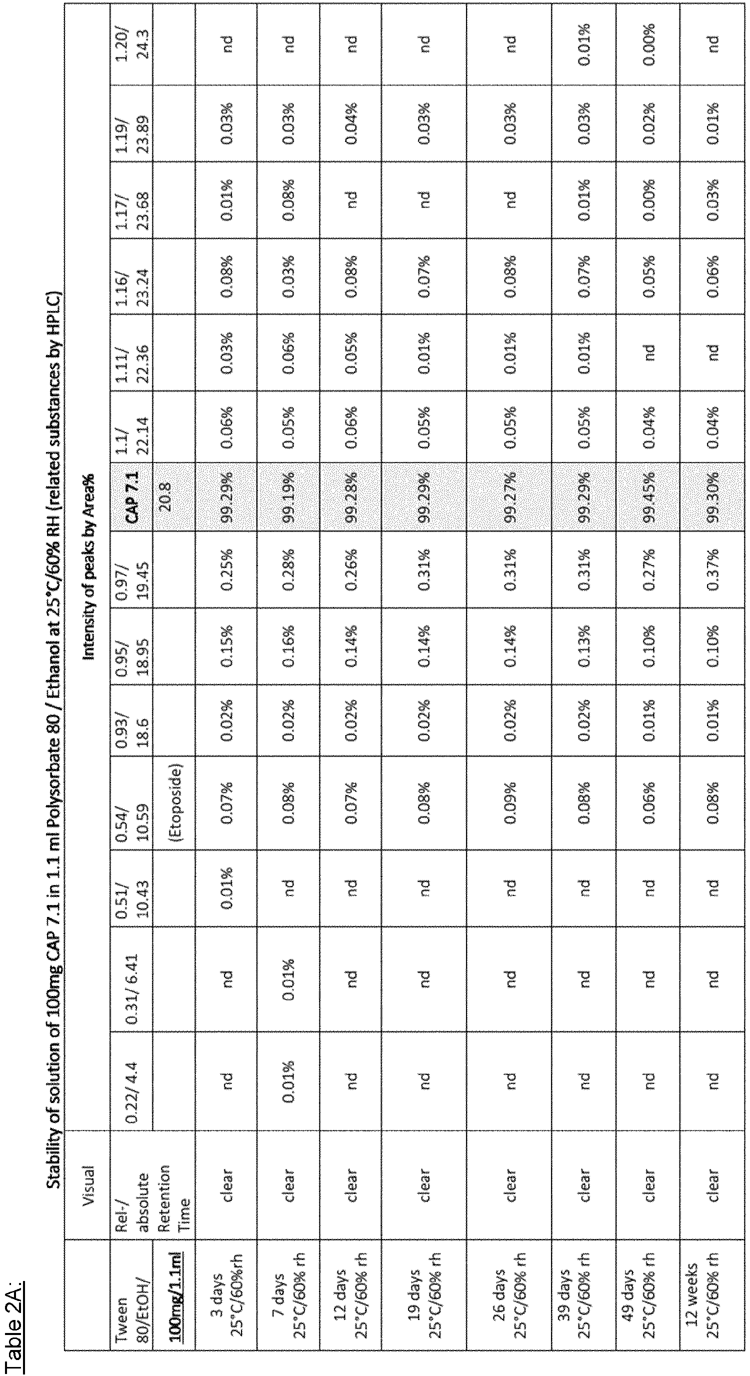

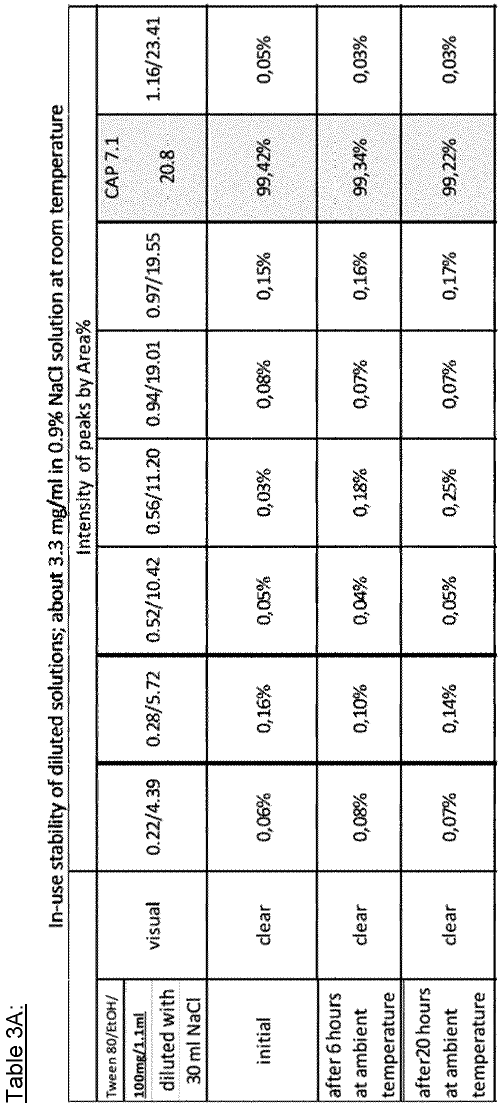

Table 4A a and 4A b:

**Table 4A a Stability of solution of 200mg CAP 7.1 in 4.0 ml Polysorbate 80 / Ethanol up to 10 weeks**

| | | | | | | | | | | | | |
|---|---|---|---|---|---|---|---|---|---|---|---|---|
| Batch F347 | | | Date of manufacturing: 02.08.2018 | | | | | Date of storage: 03.08.2018 | | | | |

| **Parameter** *(Specification)* | **Initial value** | **Storage conditions** [°C/ % RH] | **1 week** | **2 weeks** | **3 weeks** | **4 weeks** | **5 weeks** | **6 weeks** | **7 weeks** | **8 weeks** | **9 weeks** | **10 weeks** |
|---|---|---|---|---|---|---|---|---|---|---|---|---|
| **HPLC Purity Data area%** | 99.55% | KS | 99.63% | 99.57% | 99.52% | 99.55% | 99.50% | 99.51% | 99.53% | 99.53% | 99.50% | 99.52% |
| | | 25/60 | 99.57% | 99.56% | 99.50% | 99.45% | 99.50% | 99.44% | 99.50% | 99.50% | 99.48% | 99.47% |
| | | 30 / 65 | 99.55% | 99.58% | 99.49% | 99.50% | 99.48% | 99.41% | 99.46% | 99.46% | 99.41% | 99.42% |
| | | 40 / 75 | 99.56% | 99.55% | 99.42% | 99.54% | 99.31% | 99.35% | 99.23% | 99.23% | 99.20% | 99.19% |

**Table 4A b Stability of solution of 200mg CAP 7.1 in 4.0 ml Polysorbate 80 / Ethanol up to 90 days (12 weeks)**

| Batch F347 | | Date of manufacturing: 02.08.2018 | | | | | Date of storage: 03.08.2018 | | | | | |
|---|---|---|---|---|---|---|---|---|---|---|---|---|
| **Parameter** *(Specification)* | **Initial value** | **Storage conditions** [°C/ % RH] | **90 days** | | | | | | | | | |
| **HPLC Purity Data area%** | 99.55% | KS | 99.53% | | | | | | | | | |
| | | 25 / 60 | 99.49% | | | | | | | | | |
| | | 30/65 | 99.40% | | | | | | | | | |
| | | 40/75 | 99.06% | | | | | | | | | |

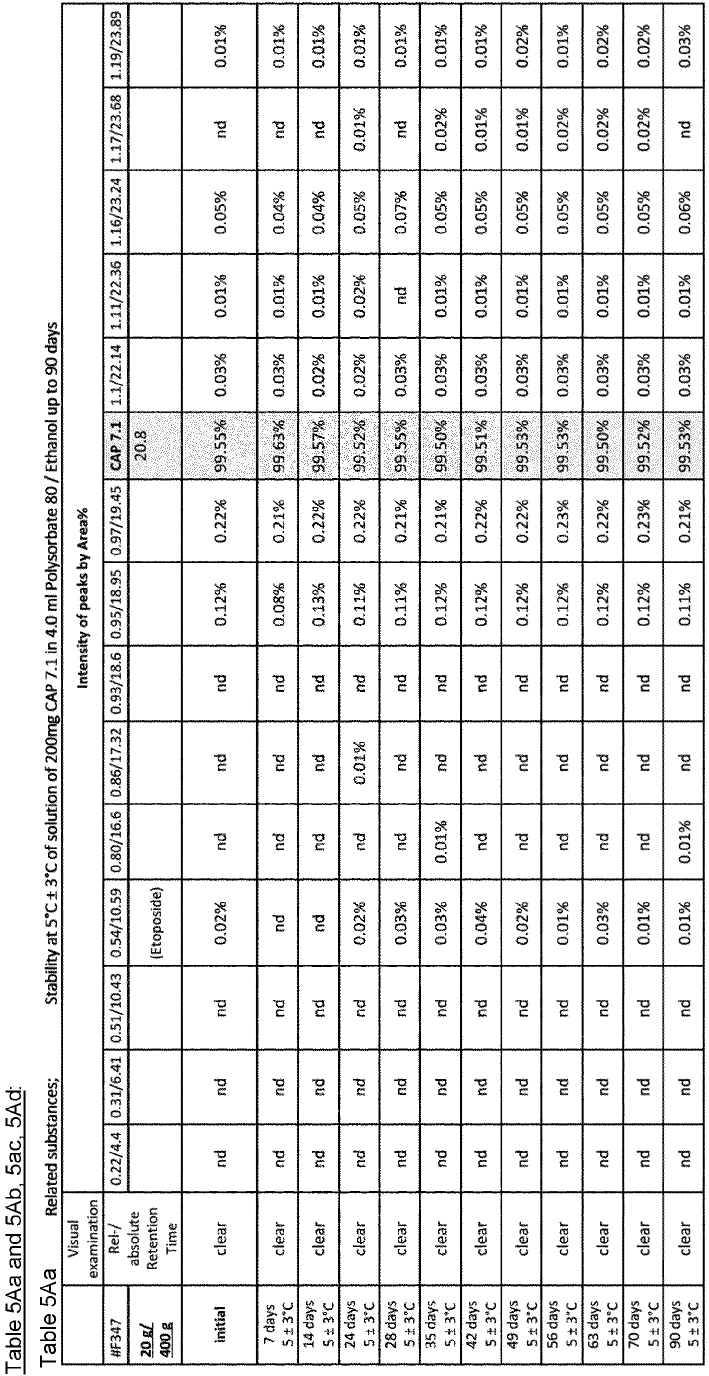

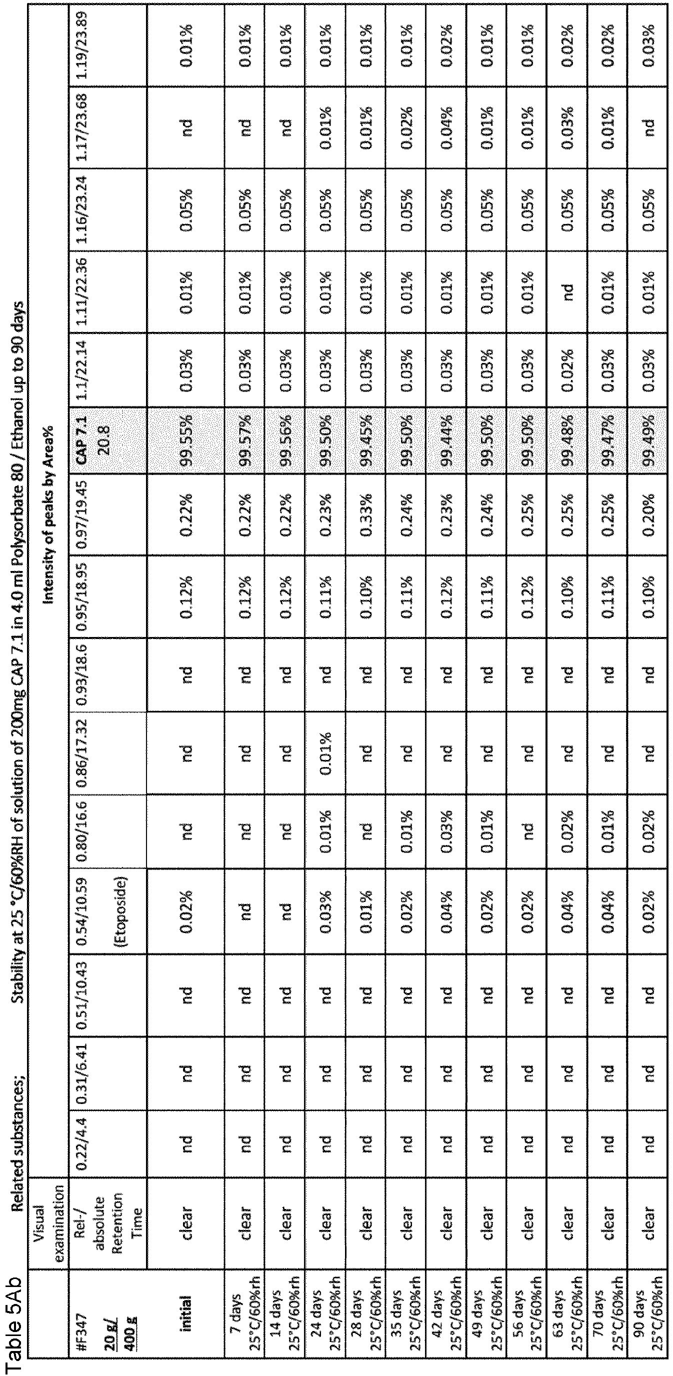

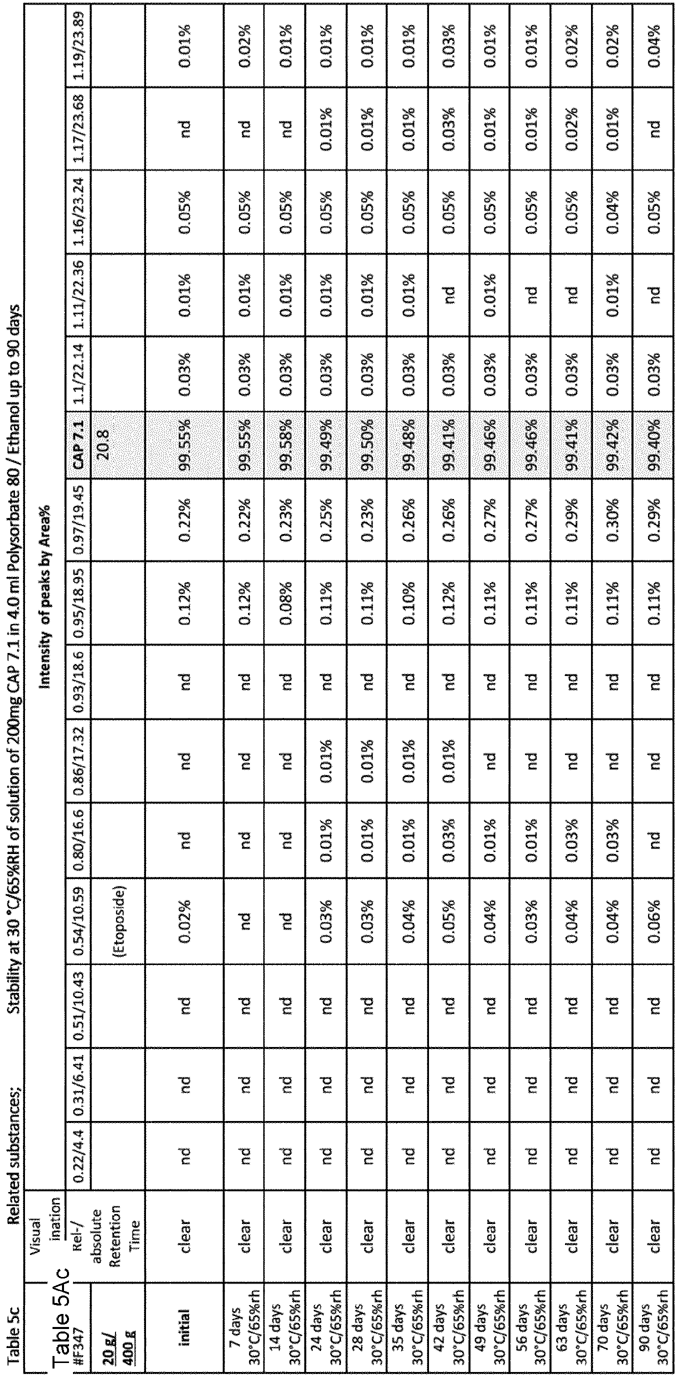

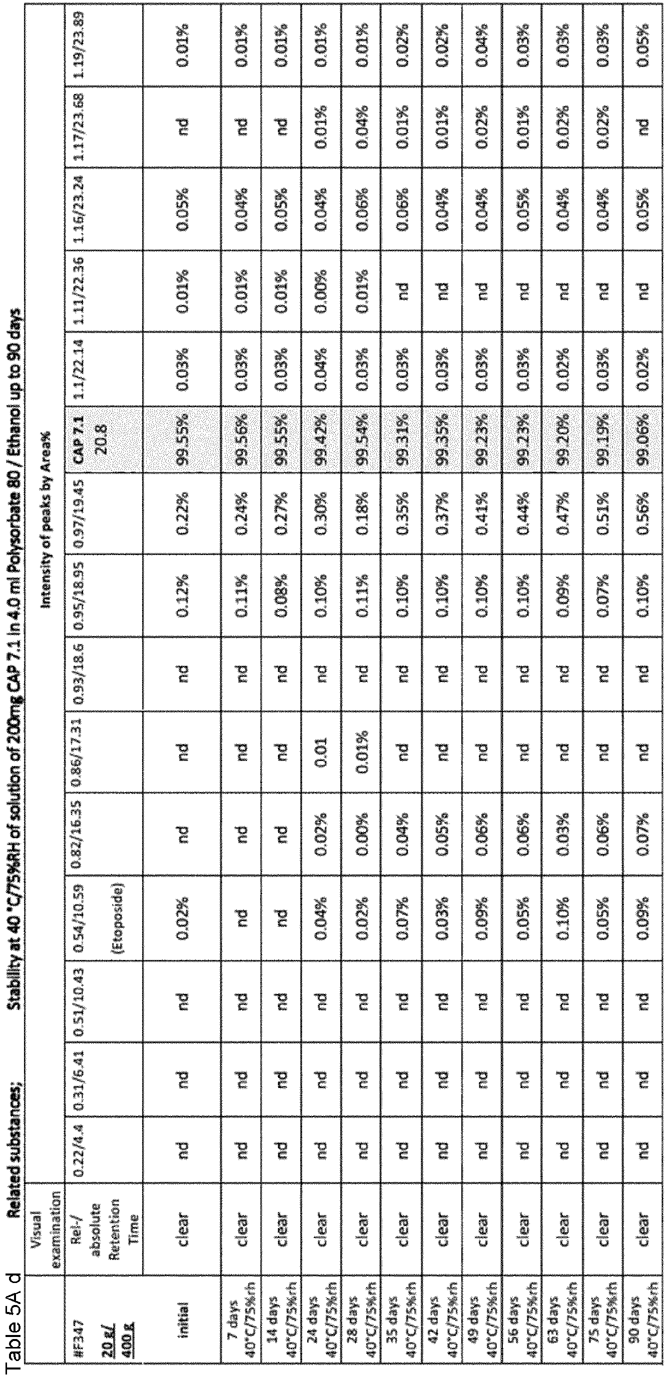

**Table 6A:**

| Sample dilution | Chemical Stability Rel. area% of CAP 7.1 peak | | | Physical Stability Sub visible particles Counts/ml [≥ 10µm / ≥ 25µm) | |
|---|---|---|---|---|---|
| | Initial | After 5 hours | After 24 hours | initial | After 24 hours |
| Water for injection | 99.48 % Etoposide 0.06 % | 99.39 % Etoposide 0.17% | 99.30 % Etoposide 0.27% | 2/0 | 2/0 |
| 0.45% NaCl solution | 99.53 % Etoposide 0.04 % | 99.38 % Etoposide 0.19 % | 99.27 % Etoposide 0.30 % | 2/0 | 2/0 |
| 0.9% NaCl solution | 99.58 % Etoposide 0.04 % | 99.37 % Etoposide 0.20 % | 99.23 % Etoposide 0.33 % | 2/0 | 1/0 |
| 5% Glucose solution | 99.52 % Etoposide 0.04 % | 99.41 % Etoposide 0.15 % | 99.30 % Etoposide 0.27 % | 3/0 | 1/0 |

**Table 7A:**

| Sample description | Storage condition | Initial value Assay [Area%] | Assay after irradiation [Area%] |
|---|---|---|---|
| #1901001 | unwrapped | 99.20 | 99.18 |
| #1901001 | in cardboard box | | 99.19 |
| #1901001 | Wrapped in aluminium foil | | 99.18 |

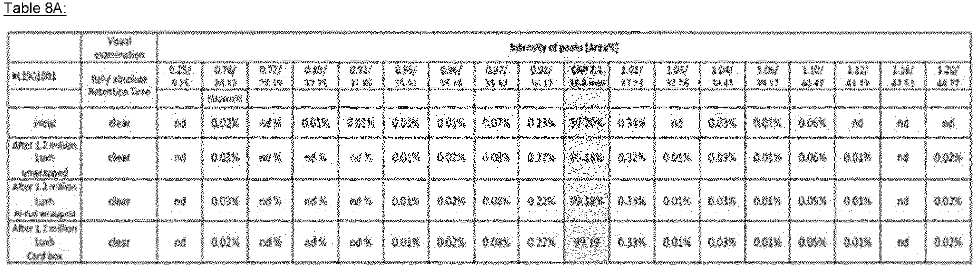

**Table 9A:**

| Retention Time / Storage time | RRT:0,51 RT:10,43 | RRT: 0,54 RT: 10,89 (Etoposide) | RRT: 0,95 RT: 19,13 | RRT: 0,97 RT: 19,67 | CAP 7.1 RT: 20,25 | RRT: 1,10 RT: 22,32 | RRT: 1,15 RT: 23,46 | RRT: 1,19 RT: 24,11 |
|---|---|---|---|---|---|---|---|---|
| initial | nd | 0,10 % | 0,10 % | 0,48 % | 99,21 % | 0,05 % | 0,04 % | 0,03 % |
| 5 days | 0,09 % | 0,17 % | 0,09 % | 0,67 % | 98,83 % | 0,04% | 0,07 % | 0,04 % |
| 12 days | 0,09 % | 0,22 % | 0,09 % | 0,87 % | 98,58 % | 0,03 % | 0,07 % | 0,04% |
| 17 days | 0,17 % | 0,33 % | 0,10 % | 1,10 % | 98,18 % | 0,04% | 0,04 % | 0,04% |

### Example 5

Etoposide toniribate as API was known to be poorly soluble in water, and unstable at acidic and basic pH. To date, the API has been formulated by dissolving it in Cremophor RH40 and ethanol. However, this formulation is cumbersome to use and lacks scalability. Experiments were undertaken to develop a new formulation of etoposide toniribate that is stable at refrigerator temperature (2-8°C) and compatible with acceptable infusion diluents, such that the infusion solution is stable for at least 2-3 hours at room temperature (assessed at controlled room temperature of 20-25°C, 60% relative humidity (RH)). It is also desirable for the infusion solution to have an osmolality close to blood osmolality (280-295 mOsm/Kg).

### Test 1

A 5 ml solution was prepared according to the following amounts:
Composition (5 ml)

| | |
|---|---|
| CAP 7.1 | 100 mg |
| PEG 300 | 3.250 mg |
| Polysorbate 80 | 400 mg |
| Ethanol | 1.51 ml |
| Benzyl alcohol | 0.30 ml |

The order of addition of the materials was as follows: CAP 7.1, ethanol, benzyl alcohol, PEG 300 and polysorbate 80. Initially stirred at 700 rpm until the addition of PEG 300 when the speed was increased to 1000 rpm for 1 hour. The formulation was stored in a refrigerator (2-8°C) for 72 hours.

RESULTS: The samples were observed after 72 hours in the refrigerator. No precipitate occurred.

### Test 2 (Batch PFD019/01)

The formulation was prepared according to the following:
Composition/10 ml

| | |
|---|---|
| CAP 7.1 | 200 mg |
| PEG 300 | 0.800 g |
| Polysorbate 80 | 6.500 g |
| Ethanol | 2.42 g |
| Benzyl alcohol | 0.31 g |

The order of addition and the manufacturing process were the same as in the previous test: CAP 7.1, ethanol, benzyl alcohol, PEG 300 and polysorbate 80. Initially stirred at 700 rpm until the addition of PEG 300 when the speed was increased to 1000 rpm for 1 hour. The formulation was stored in a refrigerator (2-8°C) for 72 hours.

RESULTS: The samples were observed after 72 hours in the refrigerator. No precipitate occurred. The formulation had a pH of 7.8.

By analysis, a concentration of 20.0 mg/ml (theoretical 20 mg/ml) was obtained after verification of the analytical method, evaluating linearity and recovery in the range 0.1 - 0.0001 mg/ml.

### Test 3

The formulation was prepared according to the following:
Composition/40 ml

| | |
|---|---|
| CAP 7.1 | 400 mg |
| PEG 300 | 400 mg |
| Polysorbate 80 | 3.2 g |
| Ethanol | 9.7 g |
| Benzyl alcohol | 1.25 g |

The order of addition of the materials was as follows: CAP 7.1, ethanol, benzyl alcohol, PEG 300 and polysorbate 80. Initially stirred at 700 rpm until the addition of PEG 300 when the speed was increased to 1000 rpm for 1 hour.

Preparation of 0.9% saline solution:

| | |
|---|---|
| NaCl | 4.5g |
| Water for injection | 500 ml |

Dissolve NaCl in the water for injection. Stir at 910 rpm until dissolved. Add the previously prepared CAP 7.1 solution to the saline solution. Stirring continued at 910 rpm for 5 minutes.

From analysis of the saline solution, a concentration of 0.82 mg/ml was obtained. A final pH of the solution of 7.23 was obtained.

**RESULTS**: Below is a summary table describing the appearance of the CAP 7.1 solution in saline stored at 2-8°C.

| **Day** | **Day 1** | | | | | **Day 2** | **Day 3** |
|---|---|---|---|---|---|---|---|
| **Time** | 13:30 | 15:00 | 16:00 | 17:00 | 18:00 | 8:00 | 7:00 |
| **Preparation Time** | 0 | 1 h 30 min | 2 h 30 min | 3 h 30 min | 4 h 30 min | 18 h 30 min | 41 h 30 min |
| **Appearance*** | C | C | C | C | C | C | C |

| | | | | | | | |
|---|---|---|---|---|---|---|---|
| *(***) Appearance clear solution with no particles. It will be Compliant (C) if this criterion is met, or Non-Compliant (NC) if it is not met.* | | | | | | | |

It can be concluded that the appearance of the CAP 7.1 solution was stable in 0.9% saline at least up to 41 hours.

### Test 4

The formulation was prepared according to the following:
Composition/ 10ml

| | |
|---|---|
| CAP 7.1 | 100 mg |
| PEG 300 | 6.50 g |
| Polysorbate 80 | 0.80 g |
| Dehydrate alcohol | 2.42 g |
| Benzyl alcohol | 0.31 g |

A batch size of 1,450 ml was made, and vials filled with a volume of 10 ml.

The order of addition of the materials was as follows: CAP 7.1, ethanol, benzyl alcohol, PEG 300 and polysorbate 80. Initially stirred at 700 rpm until the addition of PEG 300 when the speed was increased to 1000 rpm for 1 hour. A final pH of the solution of 7.26 and a density of 1.017 g/ml were obtained.

RESULTS: The results of this study are shown in Tables 1B-3B below. From the results obtained for this pre-stability (I) of CAP 7.1 solution for injection, it was concluded that it was not stable at room temperature (20-25°C/60% RH). The impurity Cis-CAP 7.1 increases at 2 months from 0.18% to 1.14%, while unknown impurities (rrt: 0.28 and rrt: 0.95) remained constant, so it was decided to stop the pre-stabilities at room temperature at the 2M time point.

However, the formulation was stable under refrigerator conditions (2-8°C). The degradation of the CAP 7.1 solution was lower, since the impurity Cis-CAP 7.1 only increased from 0.18% to 0.65% at the 6M time point.

**Table 1B:**

| | | | | | | | | |
|---|---|---|---|---|---|---|---|---|
| **ASSAY** | | | | | | | | |
| | | | | | | | | |

| Time | Concentration (mg/mL) | | | | | | | |
|---|---|---|---|---|---|---|---|---|
| **Bulk (t = 0)** | **10.02** | | | | | | | |
| | Temperature 2-8 °C/N₂ | | Temperature 2-8 °C/O₂ | | Temperature 25 °C/N₂ | | Temperature 25 °C/O₂ | |
| | V | I | V | I | V | I | V | I |
| | C (mg/mL) / % RSD | C (mg/mL) / % RSD | C (mg/mL) / % RSD | C (mg/mL) / % RSD | C (mg/mL) / % RSD | C (mg/mL) / % RSD | C (mg/mL) / % RSD | C (mg/mL) / % RSD |
| **1 week** | **9,95 / 0,4** | **9,64 / 4,3** | **9,91 / 0.1** | **9,91 / 0,6** | **9,96 / 0,6** | **9,67 / 2,6** | **9,94 / 0,9** | **9,85 / 0,6** |
| **2 weeks** | **9,71 / 0,2** | **9,70 / 0,9** | **9,80 / 1,1** | **9.67 / 0.0** | **9,67 / 0,4** | **9,55 / 2,3** | **9,66 / 0,0** | **9,71 / 0,3** |
| **3 weeks** | **10,16 / 0,4** | **10,16 / 0,4** | **10,03 / 0,8** | **10,16 / 0,1** | **10,06 / 1,1** | **10,03 / 0,8** | **10,05 / 0,8** | **10,02 / 0,2** |
| **5 weeks** | **9,96 / 0,3** | **10,05 / 0,7** | **10.03 / 0.3** | **10,00 / 0,2** | **10,00 / 0,4** | **9,98 / 0,9** | **9,88 / 0,2** | **9,94 / 0,1** |
| **1.5 months** | **10,16 / 0,9** | **10.13 / 0.2** | **9.96 / 0.7** | **10.10 / 0.1** | **9.97 / 0.3** | **10.03 / 0.4** | **9.98 / 0.6** | **9.95 / 0.7** |
| **2 months** | **9.93 / 0.3** | **9.89 / 1.7** | **9.98 / 0.4** | **9.82 / 1.7** | **9.59 / -** | **9.83 / 1.2** | **9.89 / 0.6** | **9.76 / 2.1** |
| **3 months** | **10.07 / 0.6** | - | **10.06 / 0.9** | - | **-** | - | - | - |
| **4 months** | **-** | **-** | - | **9.86 / 0.2** | **-** | **-** | **-** | **-** |
| **6 months** | **-** | **9.87 / 0.2** | - | **-** | | **-** | **-** | **-** |

**Table 2Ba (SSRR):**

| Time | **Impurity** | **RRT** | **% Impurity** |
|---|---|---|---|
| Bulk (t = 0) | Impurity Cis-CAP7.1 | 0.98 | **0.18** |
| | Unknown impurity | 0.28 | **0.12** |
| | Unknown impurity | 0.95 | **<LOQ** |
| | Totals | | **0.3** |

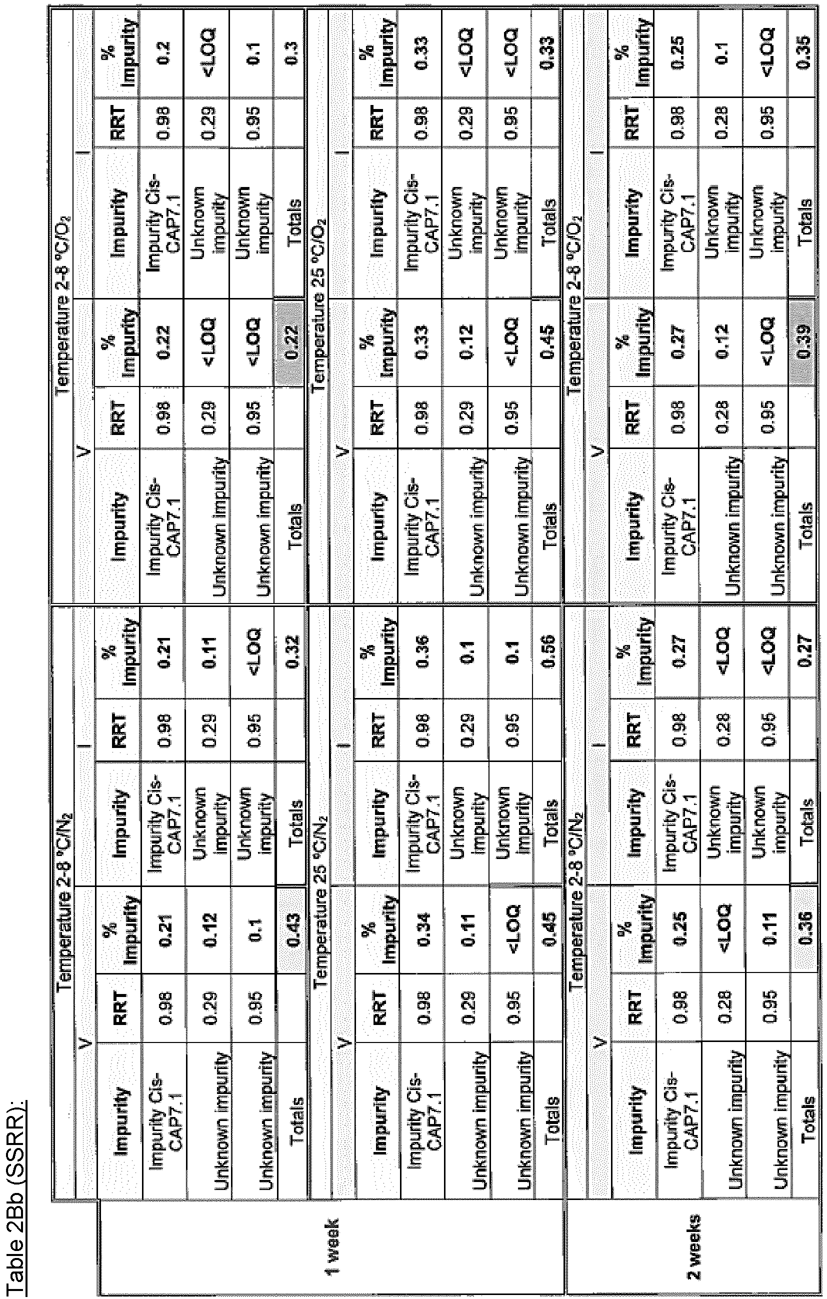

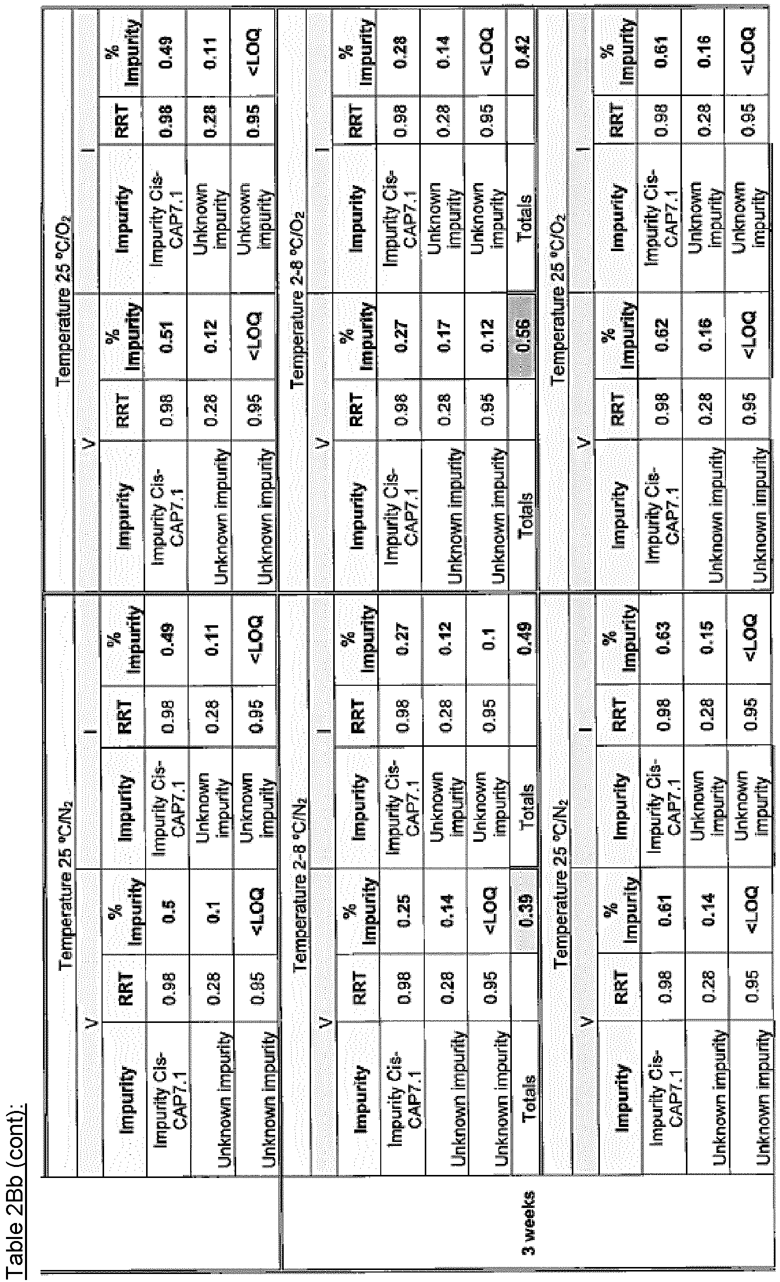

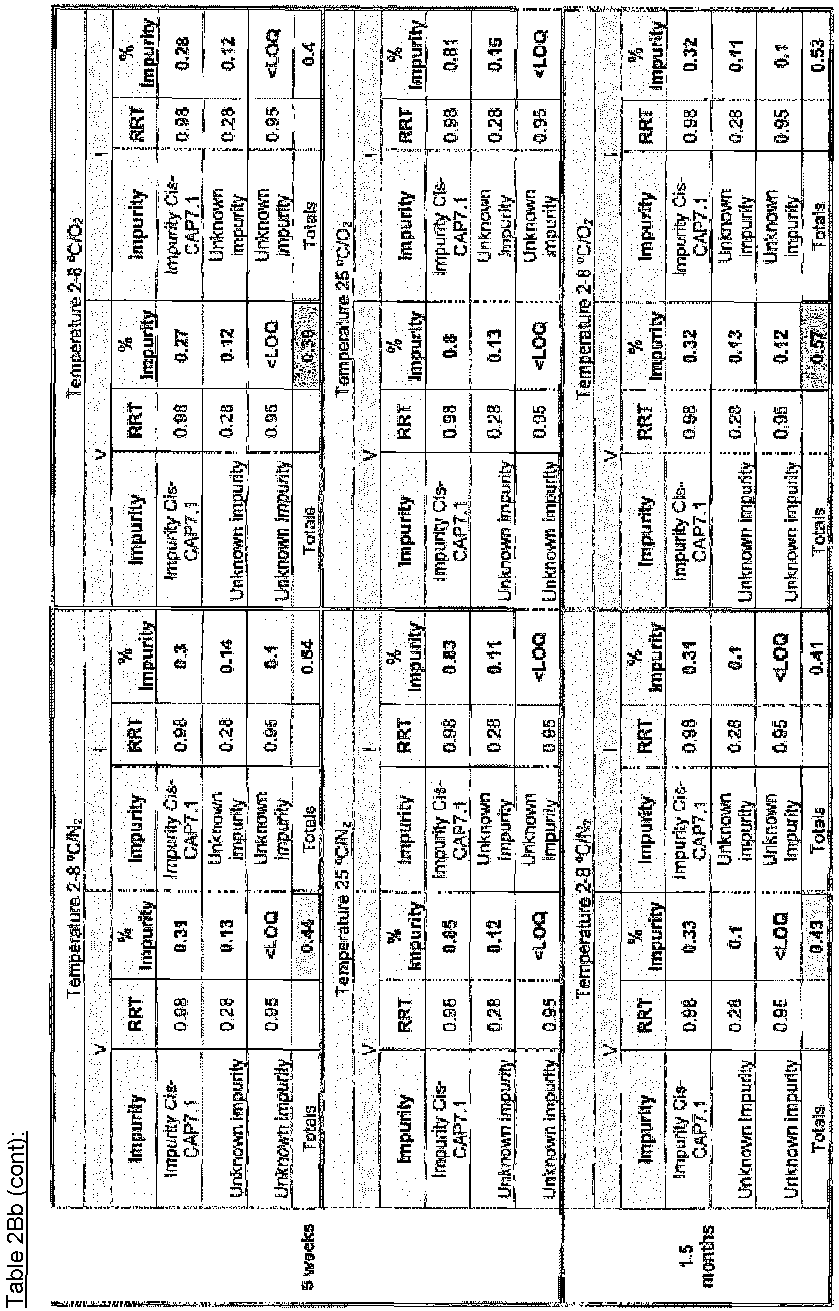

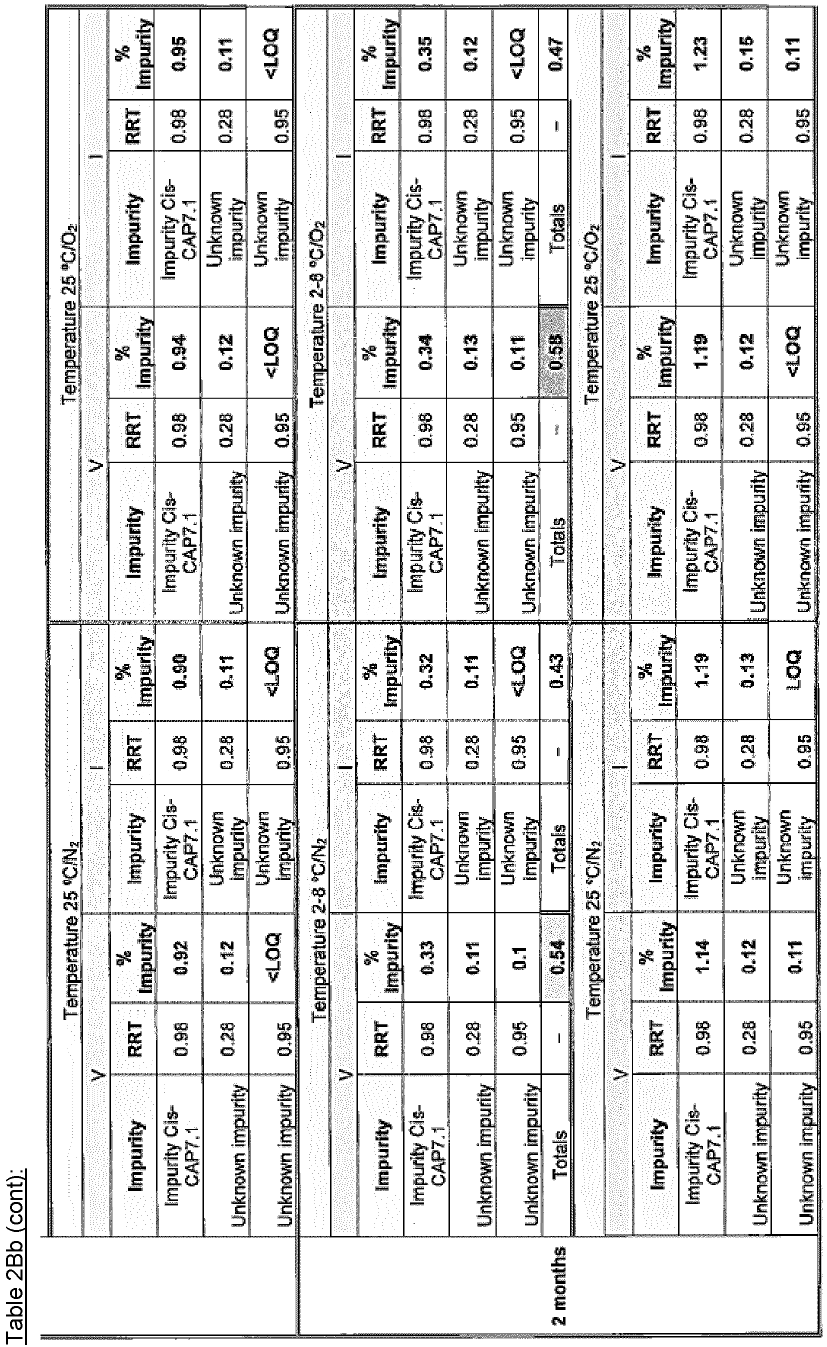

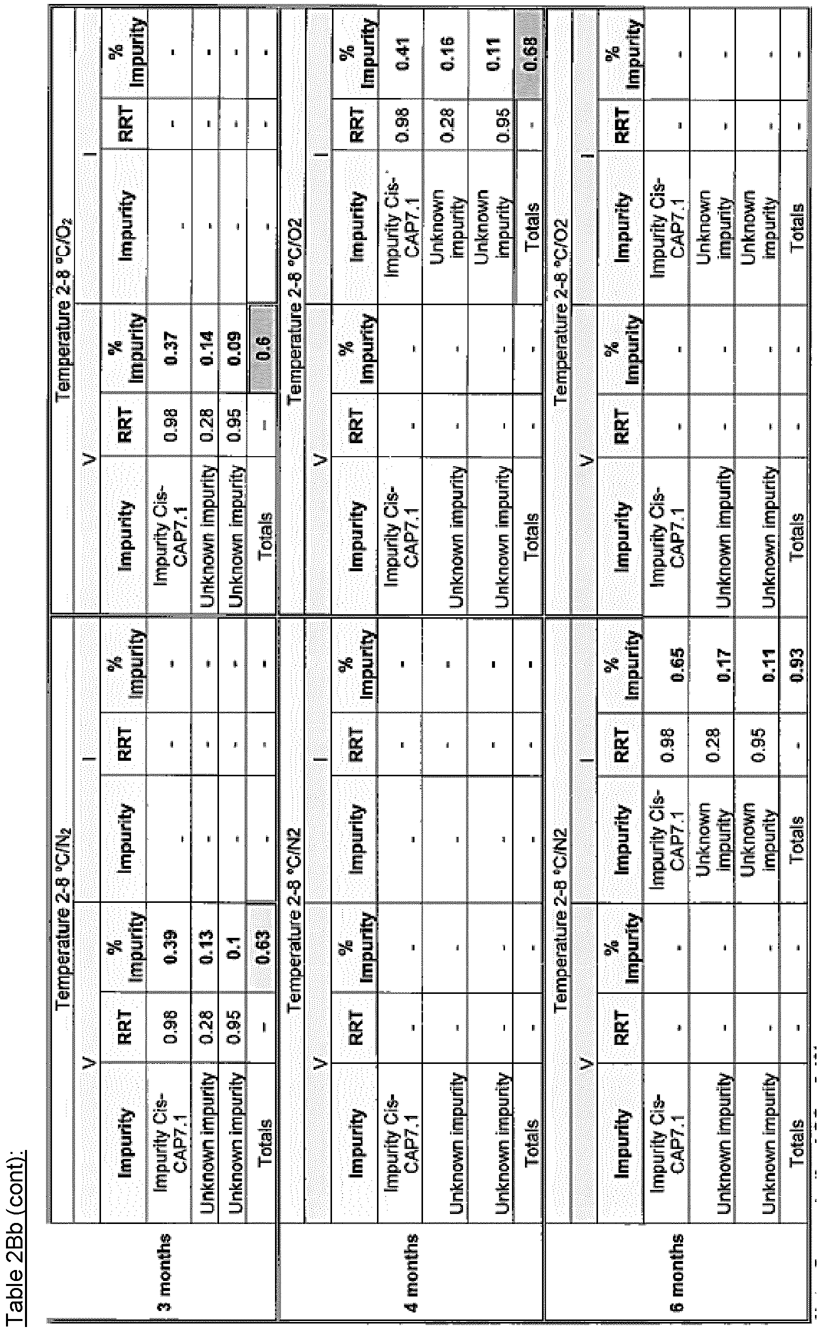

**Table 3B (pH):**

| Time | pH | | | | | | | |
|---|---|---|---|---|---|---|---|---|
| **Bulk (t = 0)** | **7.26** | | | | | | | |
| | Temperature 2-8 °C/N₂ | | Temperature 2-8 °C/O₂ | | Temperature 25 °C/N₂ | | Temperature 25 °C/O₂ | |
| | V | I | V | I | V | I | V | I |
| | pH | pH | pH | pH | pH | pH | pH | pH |
| **1 week** | **7.53** | **7.54** | **7.56** | **7.59** | **7.32** | **7.54** | **7.70** | **7.48** |
| **2 weeks** | **7.80** | **7.68** | **7.81** | **7.60** | **7.70** | **7.68** | **7.23** | **7.66** |
| **3 weeks** | **7.70** | **7.52** | **7.69** | **7.72** | **7.69** | **7.70** | **7.79** | **7.69** |
| **5 weeks** | **8.02** | **8.03** | **8.01** | **8.02** | **7.90** | **7.95** | **8.01** | **8.01** |
| **1 month 1/2** | **7.63** | **7.85** | **7.59** | **7.60** | **7.88** | **7.64** | **7.65** | **7.50** |
| **2 months** | **7.86** | **7.73** | **7.86** | **7.78** | **7.62** | **7.60** | **7.98** | **7.49** |
| **3 months** | **7.46** | | **7.88** | - | **-** | **-** | **-** | **-** |
| **4 months** | - | **-** | **-** | **7.99** | - | **-** | **-** | **-** |
| **6 months** | - | **7.77** | **-** | **-** | **-** | - | **-** | **-** |

### Test 5

The objective was to determine the stability of the formulation dissolved in 5% glucose solution for perfusion. The formulation solution was prepared with a concentration of 10 mg/ml as set out below and vials filled with a volume of 40 ml/vial. The formulation was subsequently dissolved in 500 ml of 5% glucose solution to obtain an in bag concentration of CAP 7.1 of 0.8 mg/ml.

The formulation was prepared according to the following:
Composition/ 40ml

| | |
|---|---|
| CAP 7.1 | 400 mg |
| PEG 300 | 26 g |
| Polysorbate 80 | 3.2 g |
| Ethanol | 9.7 g |
| Benzyl alcohol | 1.258 g |

The order of addition of the materials was as follows: CAP 7.1, ethanol, benzyl alcohol, PEG 300 and polysorbate 80. The mixture was stirred at 700 rpm until the addition of PEG 300 when the speed was increased to 1000 rpm for 1 hour.

5% glucose serum was prepared from 25g glucose and 500ml water for injection.

The previously prepared CAP 7.1 solution was added to the 5% glucose solution to obtain a CAP7.1 concentration of 0.8 mg/ml. Stirring continued at 910 rpm for 5 minutes.

Once the test was finished, the solution was kept at room temperature (20-25°C/60% RH) and the appearance of the solution checked for 24 hours.

**RESULTS:** Below is a summary table showing the appearance of the CAP 7.1 solution in 5% glucose solution.

| **Day** | **Day 1** | | | | | | **Day 2** | | | |
|---|---|---|---|---|---|---|---|---|---|---|
| **Time** | **17:00** | **18:00** | **19:00** | **20:00** | **21:00** | **22:00** | **7:00** | **8:00** | **15:00** | **17:00** |
| **Preparation time** | 0 | 1h | 2h | 3h | 4h | 5h | 14h | 15h | 22h | 24h |
| **Appearance (*)** | C | C | C | C | C | C | C | C | C | C |

| | | | | | | | | | | |
|---|---|---|---|---|---|---|---|---|---|---|
| *(***) Appearance clear solution with no particles. It will be Compliant (C) if this criterion is met, or Non-Compliant (NC) if it is not met.* | | | | | | | | | | |

It can be concluded that the CAP 7.1 solution is stable in 5% glucose solution for a period of 24 hours.

### Test 6

The objective was to confirm stability by testing a new batch of formulation at two different fill volumes, and also at 3 different pH levels.

The conditions for this new pre-stability study were:
- Storage in a refrigerator (2-8°C)
- Atmosphere O₂
- Vertical position of the vial (Fluorotec^{®} cap)
- pHs: 5-6, 6-7 and 7.3-7.8 (3 different formulations)
- Sampling time points: time 0, 1 week, 3 weeks, 6 weeks, 3 months and 5 months.

The formulation was prepared to provide 2000ml of solution as follows:
Amounts
weighed

| | |
|---|---|
| CAP 7.1 | 20 g |
| PEG 300 | 1.3 g |
| Polysorbate 80 | 160 g |
| Ethanol | 484 g |
| Benzyl alcohol | 162 g |

The order of addition of the materials was as follows: CAP 7.1, ethanol, benzyl alcohol, PEG 300 and polysorbate 80. Initially stirred at 700 rpm until the addition of PEG 300 when the speed was increased to 1000 rpm for 1 hour.

From this solution the following was taken:
- 760 ml and adjusted to pH 5-6 by adding 941 mg citric acid. The final pH of the solution was 5.8 and the appearance of the solution was cloudy.
- 450 ml and adjusted to pH 6-7 by adding 294 mg citric acid. The final pH of the solution was 6.5 and the appearance of the solution was transparent.

The vials for the 3 solutions have been filled at 10 ml for the pH's 5-6 and 6-7 and 20 ml for the pH's 5-6 and 7.3-7.8.

RESULTS: The results of this study are shown in Tables 4B-6B below. In summary:
1) In the 3 formulations, a new impurity occurred (rrt: 0.28) that remained constant over time. After further investigation, it was found that this impurity came from the benzyl alcohol solvent used and was unrelated to the stability of the formulation.
2) The filling volume did not affect the analytical level. The results obtained were of the same order regardless of the filling volume.
3) The formulation with a more acidic pH tended to have a lower increase in levels of the impurity cis-CAP7.1. At the 5M time point, 0.42% was obtained with the 7.3-7.8 formulation, whereas at pH 5-6 and 6-7, it was 0.22-0.24%
4) Purity remained stable over time for the 3 formulations.
5) In the stability in use study, it can be concluded that the solution was stable for 24 h at room temperature (20-25°C/60% RH) for the two concentrations of 0.7 and 2.9 mg/ml.

The results obtained from the osmolality test performed with an infusion of 0.9% saline solution was 1040 mOsm/kg. It is preferable for infusion solutions to be closer to blood osmolality.

**Table 4B:**

| Time | Concen tration (mg/mL) | | |
|---|---|---|---|
| | pH 7.3 - 7.8 | pH 6 - 7 | pH 5 - 6 |
| **bulk (zero)** | **10.55** | **10.98** | **11.73** |
| **1 week, dosage 10 mL** | **-** | **11.02** | **11.78** |
| **1 week, dosage 20 mL** | **10.83** | **-** | **11.77** |
| **3 weeks, dosage 10 mL** | **-** | **11.34** | **11.94** |
| **3 weeks, dosage 20 mL** | **10.82** | **-** | **11.87** |
| **6 weeks, dosage 10 mL** | **-** | **11.21** | **11.9** |
| **6 weeks, dosage 20 mL** | **10.83** | **-** | **11.92** |
| **3 months, dosage 110 mL** | **-** | **11.04** | **11.77** |
| **3 months, dosage 20 mL** | **10.53** | | **11.71** |
| **5 months, dosage 10 mL** | **-** | **10.88** | **11.26** |
| **5 months, dosage 20 mL** | **10.47** | - | **11.72** |

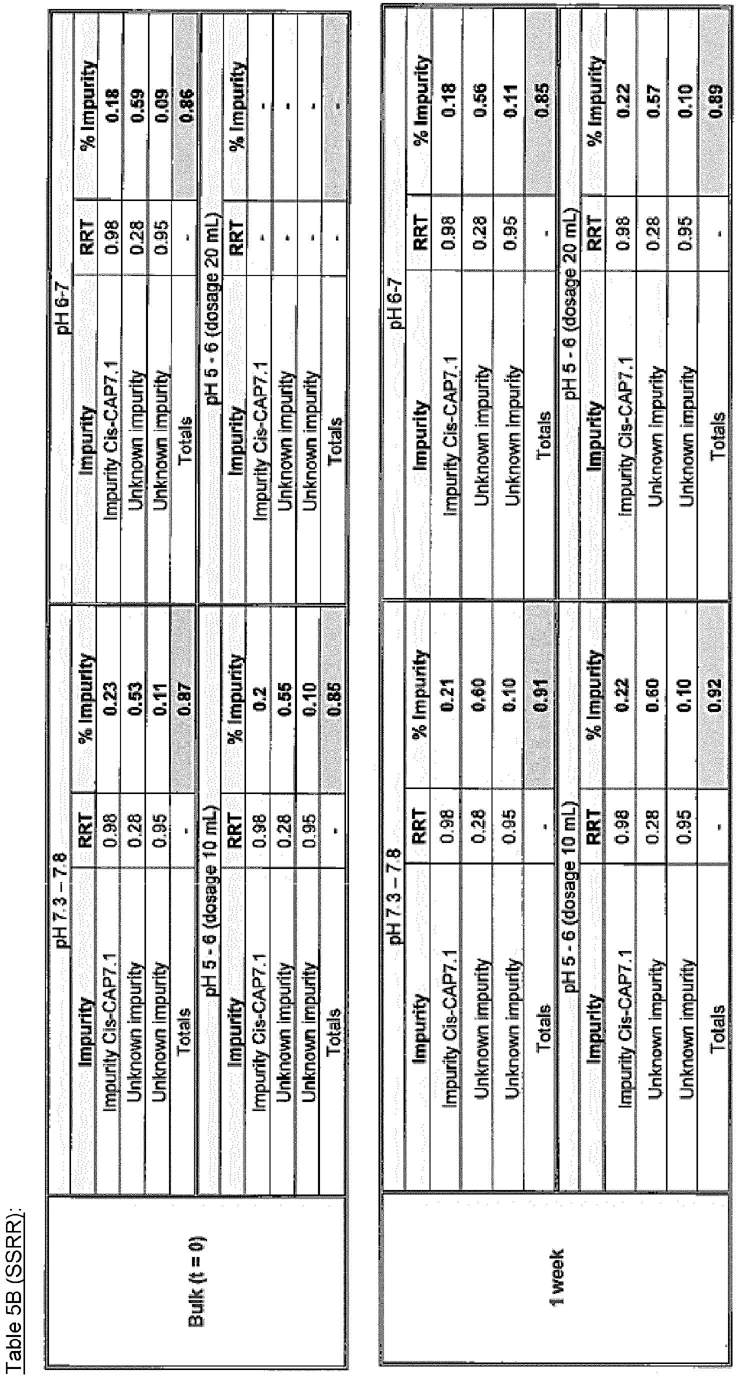

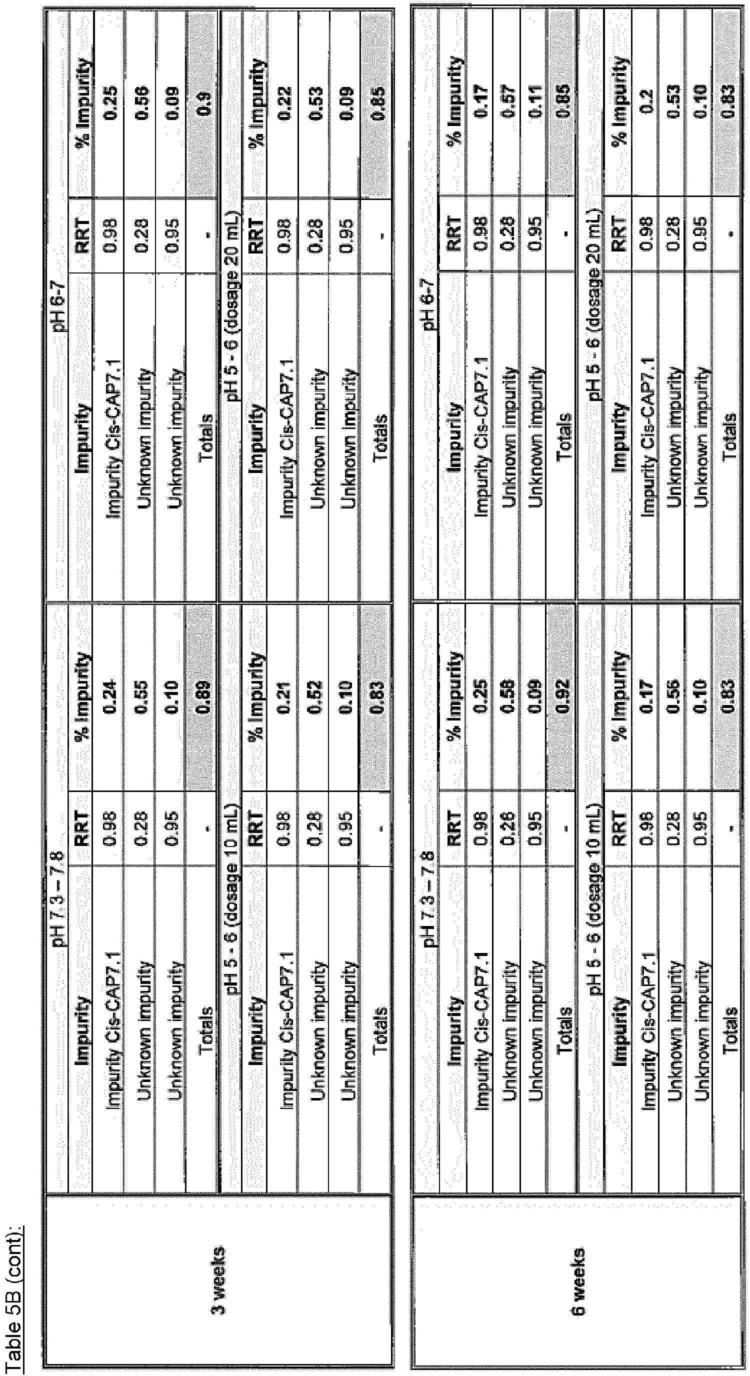

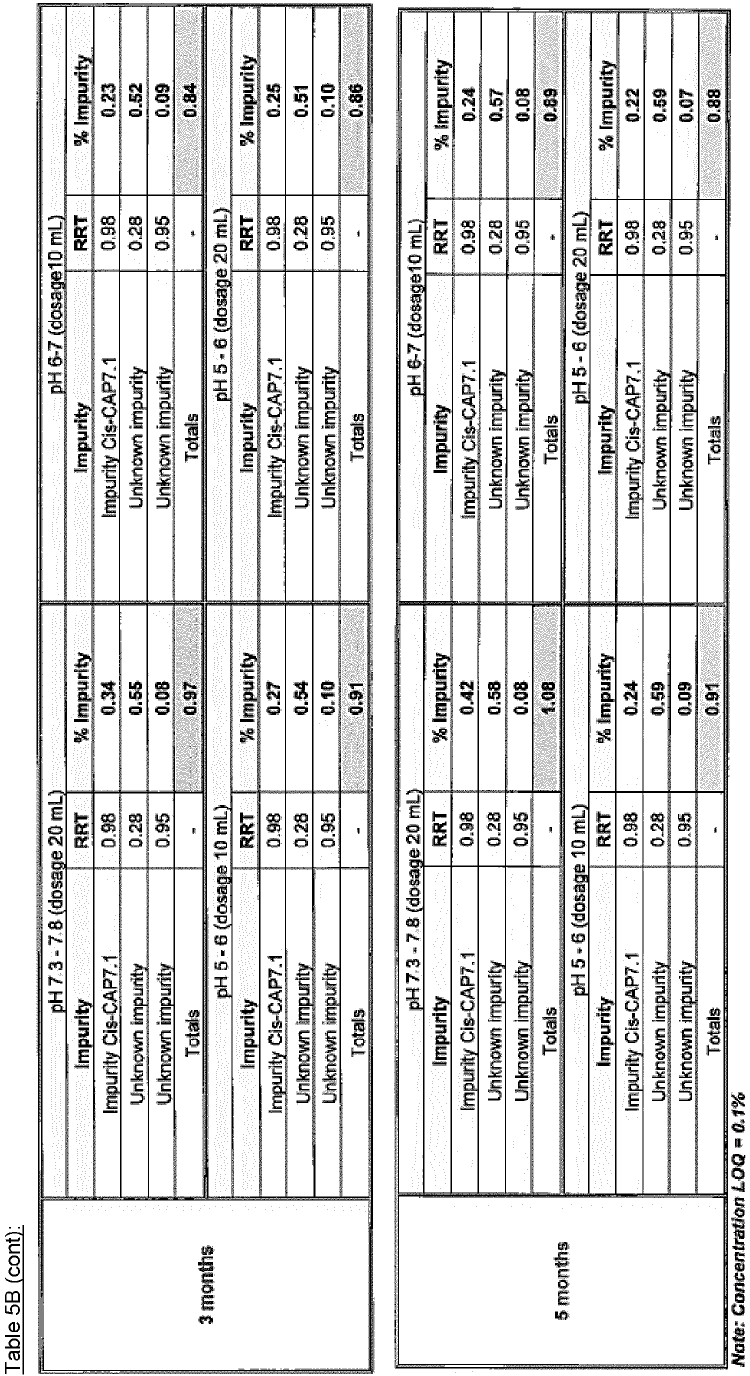

**Table 6B (pH):**

| | Temperature 2-8 °C | | |
|---|---|---|---|
| **Time** | **pH 7.3 - 7.8** | **pH 5 - 6** | **pH 6 - 7** |
| **Bulk (t** = **0)** | 7.27 | 5.2 | 6.38 |
| **1 week** | NP | NP | NP |
| **3 weeks** | 7.89 | 6.22 (10 ml) /6,06 (20 ml) | 7.03 |
| **6 weeks** | 7.73 | 6.22 (10 ml) /6-06 (20 ml) | 6.82 |
| **3 months** | 7.94 | 6.21 (10 ml) / 5.94 (20 ml) | 6.72 |
| **5 months** | 7.80 | 6.11 (10 ml) / 6.27 (20 ml) | 6.94 |

### Test 7

To confirm the results of Test 6, a new batch of formulation was prepared at a concentration of 10 mg/ml. The testing conditions were:
- Storage in a refrigerator (2-8°C).
- Atmosphere O₂
- Vertical position of the vial (Fluorotec^{®} cap)
- pHs: 5-6, 6-7 and 7.3-7.8.
- Sampling frequency: time 0, 2 months, 3 months and 5 months.

Osmolality at the concentration of 0.8 mg/ml with saline solution and water was also studied.

A total batch of 1,300 ml of solution was prepared according to the following formulation:
Amounts weighed

| | |
|---|---|
| CAP 7.1 | 13.0 g |
| PEG 300 | 845 g |
| Polysorbate 80 | 104 g |
| Ethanol | 315 g |
| Benzyl alcohol | 40.6 g |

The order of addition of the materials was as follows: CAP 7.1, ethanol, benzyl alcohol, PEG 300 and polysorbate 80. Initially stirred at 700 rpm until the addition of PEG 300 when the speed was increased to 1000 rpm for 1 hour.

The initial pH of the solution was 8.08. The following were taken from the total solution:
- 443.8 g and adjusted to a pH of 7-8 by adding 90 mg of citric acid. The final pH obtained was 7.45.
- 443.0 g and adjusted to a pH of 6-7 by adding 191 mg of citric acid. The final pH obtained was 6.45.
- 443.0 g and adjusted to a pH of 5-6 by adding 409 mg of citric acid. The final pH obtained was 5.74.

The 3 solutions at different pH were filled to 10 ml.

RESULTS: The results of the test are set out in Tables 7B-9B below. In summary:
1) Regarding impurity cis-CAP 7.1, it was observed that its increase for the 3 formulations was similar and did not exceed 0.3% after the 5M stability test.
2) The unknown impurity of rrt: 0.95 was stable over time and remained at <0.1%.

The results of the osmolality test were:
- Water infusion solution and a concentration of 0.8 mg/ml: 565 mOsm/Kg.
- Infusion solution 0.9% NaCl and a concentration of 0.8 mg/ml: 924 mOsm/Kg

It can be seen that the osmolality that most closely resembles blood is a water infusion solution.

**Table 7B:**

| Time | Concentration (mg/mL) | | |
|---|---|---|---|
| | pH 7-8 | pH 6 - 7 | pH 5 - 6 |
| **bulk (zero)** | **10.87** | **11.13** | **11.77** |
| **2 months** | **10.72** | **10.57** | **11.37** |
| **3 months** | **10.48** | **11.02** | **11.62** |
| **5 months** | **10.85** | **10.93** | **11.56** |

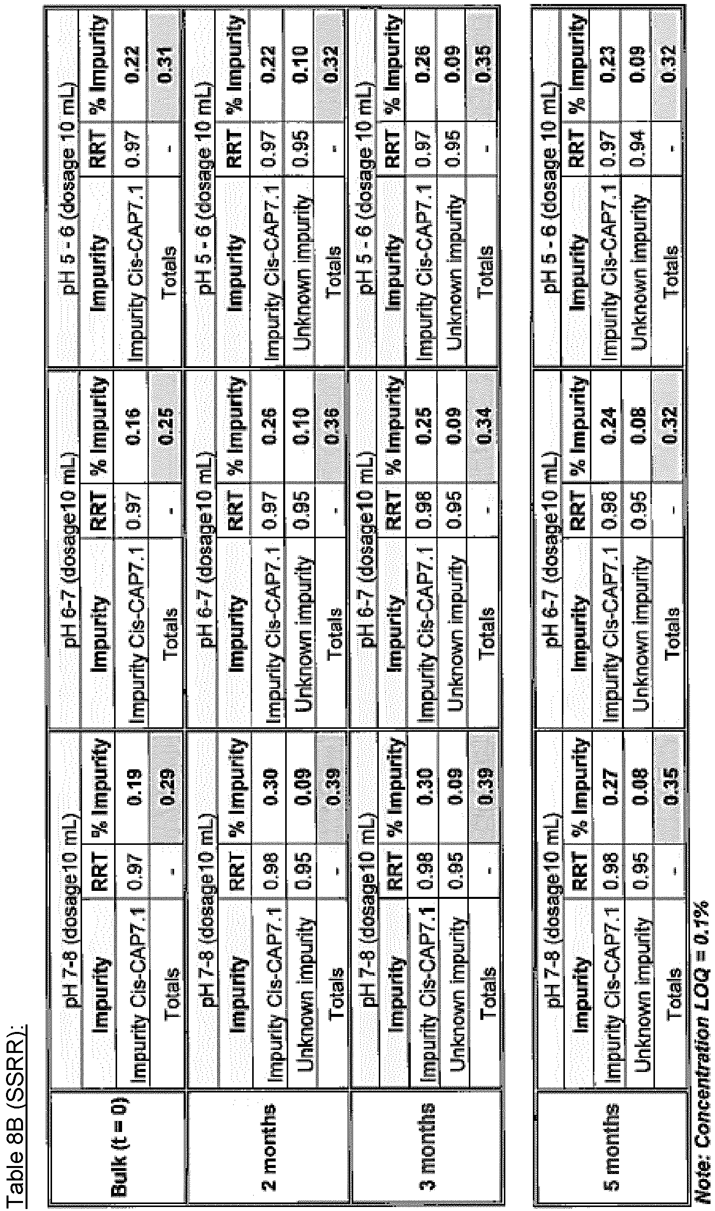

**Table 9B (pH):**

| | **Temperature 2-8 °C** | | |
|---|---|---|---|
| **Time** | **pH 7.3 - 7.8** | **pH 5 - 6** | **pH 6 - 7** |
| **Bulk (t = 0)** | 7.45 | 5.74 | 6.45 |
| **2 months** | 7.46 | 6.35 | 7.12 |
| **3 months** | 7.71 | 6.47 | 7.32 |
| **5 months** | 7.74 | 6.51 | 7.39 |

### Test 8

The objective was to assess the osmolality and stability at RT for 24 hr of a WFI infusion solution at 0.5mg/ml final concentration of CAP7.1.

A 100ml formulation was prepared according to the following:
Amounts weighed

| | |
|---|---|
| CAP 7.1 | 1.0 g |
| PEG 300 | 65.0 g |
| Polysorbate 80 | 8.0 g |
| Ethanol | 24.2 g |
| Benzyl alcohol | 3.12 g |

The formulation was diluted in water for injection to provide 800ml of an infusion solution at 0.5 mg/ml. A pH of 7.91 was obtained.

RESULTS: The osmolality test was performed at a water infusion volume of 800 ml. The result was 392 mOsm/Kg, meaning the infusion solution exhibited an osmolality close to blood osmolality.

In the stability study in use, it is concluded that the solution was stable for 24 hours at room temperature (20-25°C/60% RH) for the concentration of 0.5 mg/ml.

### Conclusions

From the tests carried out and described above, it can be concluded that:
A solution formula of CAP 7.1 with a concentration of 10 mg/ml was established. The formula is detailed as follows:
Composition/ml

| | |
|---|---|
| CAP 7.1 | 10.00 mg |
| PEG 300 | 650.0 mg |
| Polysorbate 80 | 80.00 mg |
| Ethanol | 241.60 mg |
| Benzyl alcohol | 31.20 mg |

The optimum pHs for the solution are neutral pH (7.3-7.8) and pH 6-7. To achieve pH 6-7, citric acid must be added.

At pH 5-6 it was observed that the batch solution became cloudy; however, when the solution was filled into a vial, this appearance could pass unnoticed.

Optimal storage conditions for the solution were at 2-8°C (refrigerator conditions) and in an O₂ atmosphere.

Fluorotec^{®} cap did not affect the solution, so it will be used to close the vials.

The benzyl alcohol used in the formulation should be kept under refrigerator conditions (2-8°C) and under a N₂ atmosphere.

The optimum conditions for perfusion according to the osmolality results obtained are a dose of 400 mg of API and a concentration in water of 0.5 mg/ml.
a polysorbate;
Ethanol;
Benzyl alcohol.

### Example 6: Lyophilized compositions

Etoposide toniribate is practically insoluble in water or aqueous buffer systems. However, the use of aqueous systems is mandatory for the preparation of lyophilizates. It has been found that etoposide toniribate can be dissolved in aqueous systems using ß-cyclodextrins. Suitable ß-cyclodextrins are ß**-**cyclodextrin sulfobutyl ether (Captisol^{®}), hydroxypropyl ß-cyclodextrin (Cavitron^{®}).

To suppress the hydrolysis of Etoposide toniribate in aqueous solutions, buffering of the solutions to pH values between 5.5 and 6.5 is recommended.

### Example 6-1

First, prepare a 40% solution of cavitron in a 30mM HEPES solution (HYDROXYETHYLPIPERAZINE ETHANE SULFONIC ACID SODIUM SALT). 85ml of this solution is introduced with stirring and 1.7 g of Etoposide toniribate is added. After a clear solution is obtained, it is filtered through a suitable membrane filter. The filtered solution is filled into 20ml vials at 5ml and sent for lyophilization. The vials are first cooled at a shelf temperature of -6°C until the product temperature reaches -1°C. The shelf temperature is then reduced to the lyophilization temperature. Afterwards, the temperature is reduced to - 30°C. The solution freezes at approximately -8°C product temperature. Freeze drying is performed at 0.150mbar with a slow temperature gradient of the footprint from -30°C to - 15°C over 47h. For final drying, the pressure is reduced to 0.02mbar and the footprint temperature is slowly increased to 20°C.

The resulting lyophilisates contained 100mg etoposide toniribate, 2g cavitron and 39mg HEPES. The lyophilisates could be reconstituted properly by adding 5 ml water for injection.

### Example 6-2

99.5g of 30mM HEPES buffer solution is added with stirring and 66.4g of captisol is added. The solution is adjusted to pH 6.5 with diluted diethanolamine solution (14%). 165.9 g Captisol solution is added under stirring and 1120 mg Etoposide toniribate is added. After a clear solution is obtained, the volume is adjusted to 200 ml with water. The solution is filtered through a membrane filter and filled into 50 ml vials at 15 ml corresponding to 17.85 g. The solution is freeze-dried. Freeze-drying is carried out in the same way as in example 6-1.

The resulting lyophilizates contained 84 mg etoposide toniribate, 4.98 g captisol, 117 mg HEPES-Na, and some diethanolamine from the pH adjustment.

Example 6-3 Preparation and in-use stability of an infusion solution from the lyophilizate of example 6-2.

The lyophilizates are each reconstituted with 40 ml water for injecton purposes. The stability of the reconstituted solutions was demonstrated over 17 hours by purity determination by HPLC. The data are presented in the following Table 6-3.1.

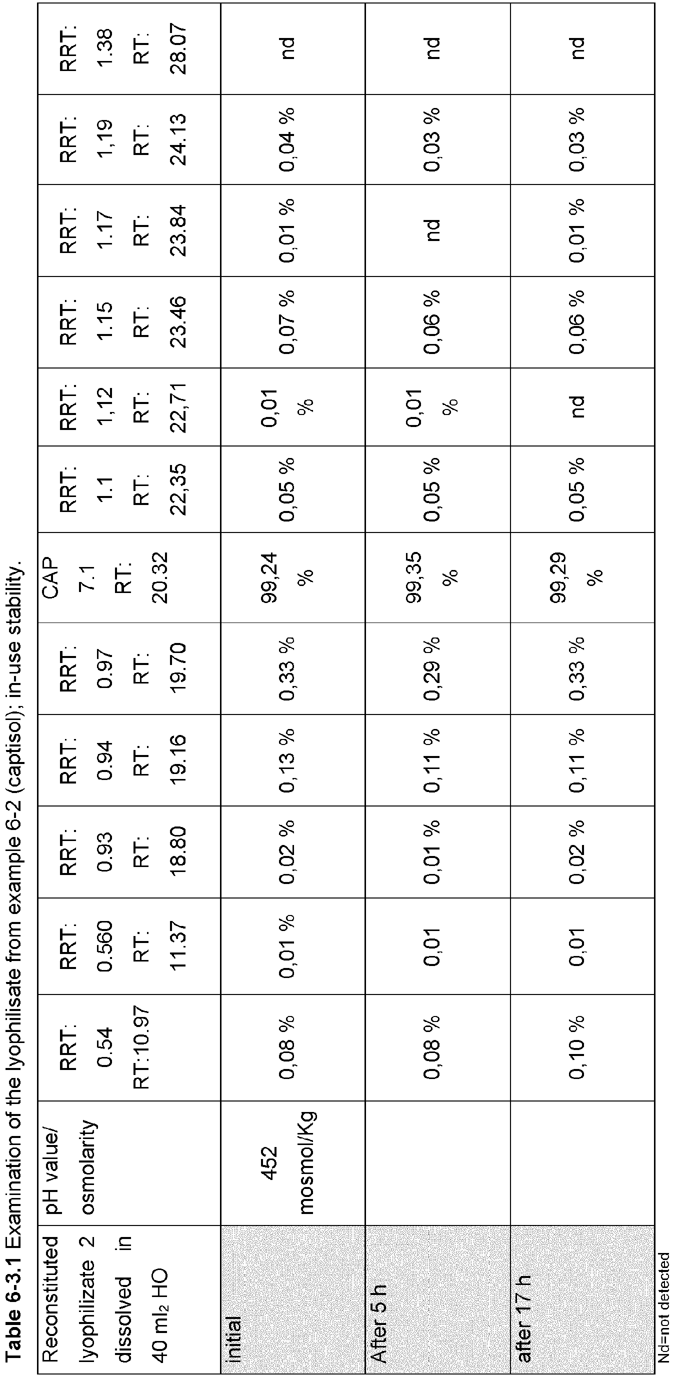

## Claims

1. **A liquid or solid pharmaceutical formulation** comprising:
etoposide toniribate;
a polysorbate;
optionally a polyethylene glycol (PEG), and
optionally, a physiological acceptable organic solvent.

2. The liquid or solid pharmaceutical formulation of claim 1, the formulation is an oral dosage form, such as a pill, tablet, caplet, hard capsule or soft capsule.

3. The liquid or solid pharmaceutical formulation of claim 1 or 2, wherein the pharmaceutical formulation is a solid formulation, and preferably in the form of a lyophilized powder, such as a lyophilized dried powder.

4. The liquid or solid pharmaceutical formulation of any one of claims 1 to 3, comprising etoposide toniribate at a concentration in the range of from 50 mg/ml to 100 mg/ml.

5. **A method of preparing an infusion solution** comprising diluting the liquid pharmaceutical formulation according to any one of claims 1-4 in a diluent; or resuspending a solid pharmaceutical formulation according to any one of claims 1-15 in a diluent.

6. **An infusion solution** prepared by diluting or resuspending a liquid or solid pharmaceutical formulation according to any of claims 1-4 in a diluent.

7. The method according to claim 5 or the infusion solution according to claim 6, wherein the diluent is selected from: water for injection; 5% glucose solution; 0.45% NaCl saline, and 0.9% NaCl saline.

8. The method or an infusion solution according to claim 7, wherein the diluent is water for injection.

9. **A kit** comprising: the liquid or solid pharmaceutical formulation of any one of claims 1-4; and a diluent.

10. **A method for producing a lyophilized composition of etoposide toniribate,** wherein the method comprises the following steps:
a. providing a liquid comprising at least etoposide toniribate and a cyclodextrin;
b. optionally filtrating the liquid;
c. cooling the liquid to a freezing temperature, wherein the cooling is performed at a defined cooling rate;
d. freezing the liquid at the freezing temperature in order to obtain a frozen liquid; and
e. drying the frozen liquid obtained in step d) in order to obtain a lyophilized composition comprising etoposide toniribate.

11. The method of claim 10, wherein the liquid in step a. is buffered at a pH between about 4 and about 7, preferably between about 5,5 and about 6,5.

12. The method of any one of claims 10 to 11, wherein cyclodextrin is beta-cyclodextrin, such as ß-Cyclodextrin Sulfobutyl ether (Captisol^{®}), Hydroxypropyl-ß**-**Cyclodextrin (Cavitron^{®}).

13. **A lyophilized composition of etoposide toniribate,** comprising etoposide toniribate and a cyclodextrin, optionally a salt.

14. **A pharmaceutical package,** comprising a lyophilized composition of etoposide toniribate according to claim 13, and a diluent for resuspending the lyophilized composition, optionally together with instructions for use.

15. **A compound or composition for use in treating cancer in a patient in need of the treatment,** wherein the treating comprises administering to the patient an effective amount of the compound or composition, and wherein the compound or composition is a liquid or solid pharmaceutical formulation according to any one of claims 1-4, or the infusion solution according to any one of claims 6-8, or a lyophilized composition of etoposide toniribate of claim 13.
